Europäisches Patentamt

European Patent Office

Office européen des brevets

(19)

(11) **EP 0 407 259 B1**

## (12) FASCICULE DE BREVET EUROPEEN

(45) Date de publication et mention
de la délivrance du brevet:
**17.01.1996 Bulletin 1996/03**

(51) Int Cl.6: **C12N 15/62**, C12N 1/21,
C12N 15/70, G01N 33/53

(21) Numéro de dépôt: **90401794.4**

(22) Date de dépôt: **25.06.1990**

(54) **Protéines hybrides entre une enzyme extracytoplasmique et au moins une autre protéine, leur procédé de préparation, ainsi que leurs applications**

Hybridproteine zwischen einem extraplasmischen Enzym und mindestens einem anderen Protein, Verfahren zur Herstellung und Anwendungen

Hybrid proteins between an extracytoplasmic enzyme and at least another protein, preparation and method and application therefor

(84) Etats contractants désignés:
**AT CH DE FR GB IT LI SE**

(30) Priorité: **26.06.1989 FR 8908444**

(43) Date de publication de la demande:
**09.01.1991 Bulletin 1991/02**

(73) Titulaire:
**COMMISSARIAT A L'ENERGIE ATOMIQUE**
**F-75015 Paris Cédex 15 (FR)**

(72) Inventeurs:
• **Boquet, Paul**
 **F-78160 Marly le Roi (FR)**
• **Boulain, Jean-Claude**
 **F-91120 Palaiseau (FR)**
• **Ducancel, Frédéric**
 **F-75012 Paris (FR)**

• **Gillet, Daniel**
 **F-75020 Paris (FR)**
• **Menez, André**
 **F-78470 St Remy les Cheuvreuse (FR)**

(74) Mandataire: **Orès, Bernard et al**
 **F-75008 Paris (FR)**

(56) Documents cités:
EP-A- 0 023 882      EP-A- 0 242 243
EP-A- 0 302 381      EP-A- 0 316 933
EP-A- 0 372 352      WO-A-86/06742

• BIOCHEMICAL AND BIOPHYSICAL RESEARCH vol. 149, no. 2, 16 December 1987, pages 607 - 614; LINDBLADH,C. ET AL.: "The design of a simple competitive ELISA using human proinsulin-alkaline phosphatase conjugates prepared by gene fusion."
• PROTEIN ENGINEERING. vol. 3, no. 2, 1989, EYNSHAM, OXFORD, ENG pages 139 - 143; DUCANCEL,F. et al.: "Direct expression in E.coli of a functinally active protein A-snake fusion protein."

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

**Description**

La présente invention est relative à des protéines hybrides entre un fragment d'une protéine P1 extracytoplasmique, une protéine P2 et une enzyme extracytoplasmique, la protéine P2 étant notamment un antigène ou un fragment variable (Fv) simple chaîne d'un anticorps, à leur procédé de préparation, ainsi qu'à leurs applications notamment comme agent de diagnostic, et dans le criblage d'une banque d'acide nucléique ou dans la sélection de clones recombinants.

La présente invention est également relative aux séquences d'ADN codant pour lesdites protéines hybrides, à des vecteurs d'expression contenant lesdites séquences, à des souches de bactéries ou de levures transformées par lesdits vecteurs ainsi qu'à leur application pour la production et la sécrétion desdites protéines hybrides.

Dans la littérature, un certain nombre de protéines hybrides ont été décrites ainsi que leur procédé de production.

On peut citer notamment les Brevets américains 4 411 994 et 4 338 397, qui décrivent l'utilisation du gène de la pénicillinase dans le plasmide pBR322 pour produire des protéines de fusion, qui sont transportées dans l'espace périplasmique par la séquence "leader" de la pénicillinase, en utilisant des séquences d'ADNc codant pour la préproinsuline, insérées au niveau du site PstI du plasmide pBR322.

On peut citer également la Demande de Brevet européen 196 864, qui décrit l'utilisation de la séquence "leader" de la phosphatase alcaline, suivie en aval et en phase de lecture, par la séquence codante pour la protéine désirée.

Le système d'expression de ladite séquence inclut un promoteur approprié (par exemple le promoteur du gène phoA), lié de manière opérationnelle à ladite séquence codante hybride et en aval de ladite séquence, un terminateur approprié, notamment le terminateur phoA.

Cette Demande de Brevet européen 196 864 fait apparaître que seules les protéines dites sensibles peuvent être fusionnées à la partie N terminale de la phosphatase alcaline et exportées ; il est, de plus, précisé qu'une protéine est considérée comme sensible lorsqu'elle comporte une séquence en amino-acides qui interagit avec la séquence leader de la phosphatase alcaline et donne lieu à la sécrétion de ladite séquence.

Il est également précisé que la sensibilité de la protéine, sans doute due à la nature de sa séquence en amino-acides qui permet cette coopération entre les deux séquences, n'est pas comprise actuellement. Il est toutefois clair que certaines protéines hétérologues sont capables d'interagir avec la séquence signal dérivée d'un certain nombre de "leader" bactériens, alors que d'autres protéines sont incapables de cette interaction.

Cette Demande européenne décrit plus particulièrement, comme protéines sensibles, l'hGH et le TNF, alors que l'IL-2 est considérée comme une protéine non sensible, c'est-à-dire non exportée. Les protéines hybrides décrites dans cette Demande, sont destinées à produire notamment de l'hGH ou du TNF en quantités industrielles, par un procédé de recombinaison génétique, la séquence signal de la phosphatase alcaline servant uniquement au transport de la protéine étrangère.

La Demande de Brevet européen 242 243 décrit une protéine hybride comprenant une enzyme contenant un épitope, inséré de telle sorte dans la chaîne peptidique de l'enzyme, que l'épitope est exposé de manière à ce que lorsque la protéine hybride est en contact immunologique avec des anticorps dirigés contre l'épitope, un complexe est formé entre ladite protéine hybride et lesdits anticorps et l'activité enzymatique de ladite enzyme est préservée soit dans l'état non complexé, soit dans l'état complexé. La protéine hybride décrite dans cette Demande européenne est repliée dans la membrane externe d'une bactérie, de telle sorte que l'épitope choisi soit exposé à la surface de la bactérie ; des souches de *E. Coli* portant à leur surface externe des récepteurs modifiés du phage lambda contenant, exposées à la surface, les protéines hybrides ci-dessus définies sont également décrites dans cette Demande. Les protéines hybrides décrites dans cette Demande, peuvent éventuellement comprendre la phosphatase alcaline, la peroxydase ou la β-galactosidase.

Il existe d'autres documents qui décrivent des protéines de fusion permettant d'obtenir le produit recherché, sécrété ou exporté. On peut citer en particulier l'article au nom de T.P. HOPP et al., paru dans Biotechnol., 1988, <u>6</u>, 1204-1210, qui décrit une séquence de fusion N-terminale courte utilisée comme marqueur pour l'identification et la purification de protéines recombinantes ; l'élimination dudit marqueur a l'avantage de ne pas nécessiter de traitements drastiques de la protéine de fusion. Il faut noter que, dans cet article T.P. HOPP et al., précisent que la réalisation de protéines de fusion présente, à l'heure actuelle, un certain nombre d'inconvénients ; en effet, il n'est pas évident d'obtenir des protéines de fusion qui présentent une structure quaternaire qui correspond à celle de la protéine native, qui soient stables et qui conservent l'activité biologique de cette dernière ; on peut également citer l'article paru dans Biochemical and Biophysical Research, 1987, **149**, 2, 607-614, qui décrit une protéine hybride qui comprend successivement la proinsuline ou un fragment de celle-ci et la phosphatase alcaline et est obtenue à partir d'un ADN comprenant successivement le gène de la proinsuline et le gène de la phosphatase alcaline (liaison par l'extrémité 5' du gène codant pour la PhoA mature).

Toutefois, les descriptions de protéines fusionnées et/ou exportées décrites dans la littérature ne s'appliquent qu'à des cas particuliers et ne peuvent être généralisables.

La Demanderesse s'est, en conséquence, donné pour but de pourvoir à une famille de protéines hybrides directement exportées dans l'espace périplasmique ou sécrétées en dehors de la cellule sous leurs formes hybrides et constituées par un fragment d'une protéine P1, une protéine P2 et une enzyme, lesdites protéines hybrides conservant les propriétés de la protéine P2 et de l'enzyme et pouvant être utilisées comme réactif de diagnostic et/ou de dépistage dans le cadre de dosages de type RA (receptor assay) ou EIA (enzyme immunoassay), en particulier de type ELISA et pour la sélection de clones recombinants ou pour le criblage d'une banque d'ADN ; de telles protéines hybrides répondent mieux aux nécessités de la pratique que les protéines hybrides de l'Art antérieur, notamment en ce que lesdites protéines hybrides exportées ou sécrétées sont directement utilisables comme réactif de diagnostic, sans étape de purification et sont stables.

De plus, de telles protéines hybrides permettent notamment le dosage de petites molécules, qui habituellement, même si elles peuvent être couplées chimiquement à une enzyme, ne peuvent pas trouver application comme réactif et qui sont, jusqu'à présent, essentiellement dosées par RIA.

C'est également un but de l'invention de fournir les outils pour la production desdites protéines hybrides.

La présente invention a pour objet une séquence hybride d'acide nucléique du type comprenant un fragment codant pour une protéine étrangère inséré dans une séquence codant pour une protéine support comprenant la séquence leader du gène de structure d'une protéine appropriée P1 exportée ou sécrétable par un microorganisme, notamment choisi dans le groupe qui comprend les bactéries et les levures, une séquence d'acide nucléique codant pour un fragment NH$_2$-terminal de ladite protéine P1 mature, un fragment codant pour au moins un fragment fonctionnel de la séquence mature d'une enzyme appropriée, laquelle séquence est caractérisée en ce qu'une séquence d'acide nucléique codant pour une protéine P2 est insérée entre ledit fragment NH$_2$-terminal de ladite protéine P1 mature et ledit fragment codant pour au moins un fragment fonctionnel de la séquence mature d'une enzyme appropriée, l'ensemble de ces fragments étant en phase de lecture et codant pour une protéine hybride présentant à la fois les propriétés de l'enzyme et certaines propriétés de ladite protéine P2, notamment celle d'interagir spécifiquement avec un anticorps, un antigène ou un récepteur.

On entend, au sens de la présente invention, par acide nucléique, aussi bien une séquence d'acide nucléique simple brin que double brin, l'acide nucléique étant aussi bien un ADN qu'un ARN.

Selon un autre mode de réalisation avantageux de la séquence conforme à l'invention, l'enzyme est choisie dans le groupe qui comprend notamment la phosphatase alcaline, la phosphatase acide, la glucose phosphatase acide, la phosphodiestérase cyclique et la β-lactamase.

Selon un autre mode de réalisation avantageux de la séquence conforme à l'invention, le fragment de séquence codant pour la protéine P2 est notamment choisi dans le groupe qui comprend les séquences codant pour les hormones peptidiques, les séquences codant pour une toxine et les séquences codant pour un fragment simple chaîne analogue aux domaines variables des immunoglobulines (immunoglobulines recombinantes) .

Ces derniers fragments sont notamment décrits dans Proc. Natl. Acad. Sci. USA, 1988, 85, 5879-5883.

Selon une disposition de ce mode de réalisation, la toxine est avantageusement une neurotoxine.

Conformément à l'invention, la protéine P1 est identique à ou différente de l'enzyme définie ci-dessus.

Selon un autre mode de réalisation avantageux de la séquence conforme à l'invention, ladite séquence d'acide nucléique est constituée par une séquence hybride comprenant successivement le fragment leader du gène de structure d'une enzyme exportée ou sécrétée par un microorganisme, notamment choisi dans le groupe qui comprend les bactéries et les levures, une séquence d'acide nucléique codant pour un fragment NH$_2$-terminal de ladite enzyme mature, une séquence d'acide nucléique codant pour une protéine P2, puis un fragment codant pour au moins un fragment fonctionnel de la séquence mature de ladite enzyme, l'ensemble de ces fragments étant en phase de lecture et codant pour une protéine hybride présentant à la fois les propriétés de l'enzyme et certaines propriétés de ladite protéine, notamment celle d'interagir spécifiquement avec un anticorps, un antigène ou un récepteur.

Selon une disposition avantageuse de ce mode de réalisation, ladite séquence hybride comprend successivement la séquence leader du gène de structure de la phosphatase alcaline, un fragment codant pour, au plus, les 28 aminoacides N-terminaux de la phosphatase alcaline mature, une séquence d'ADN codant pour une protéine P2 ou un fragment de celle-ci, puis un fragment codant pour, au moins, les 422 aminoacides restants C-terminaux de la phosphatase alcaline, l'ensemble de ces fragments étant en phase de lecture et formant une séquence d'ADN hybride codant pour une protéine hybride présentant à la fois les propriétés de la phosphatase alcaline, en particulier son activité enzymatique et certaines des propriétés de la protéine P2, notamment celle d'interagir spécifiquement avec un antigène, un anticorps ou un récepteur.

Selon une modalité avantageuse de cette disposition, ladite séquence d'ADN hybride comprend successivement la séquence d'acide nucléique codant pour le peptide leader de la phosphatase alcaline, la séquence codant pour les 28 aminoacides N-terminaux de la phosphatase alcaline, la séquence codant pour l'érabutoxine a (ea) mature, et la séquence codant pour les 422 amino-acides C-terminaux de la phosphatase alcaline.

Conformément à cette modalité, la séquence codant pour l'érabutoxine a comprend 192 paires de bases et deux sites de restriction Sau 3 Al supplémentaires.

Ladite séquence hybride comprend la séquence n° 1 ci-après :

```
                                                        Arg Thr Pro
     Fragment leader du gène de structure de la phoA-CGG ACA CCA
     Glu Met Pro Val Leu Glu Asn Arg Ala Ala Gln Gly Asp Ile Thr
     GAA ATG CCT GTT CTG GAA AAC CGG GCT GCT CAG GGC GAT ATT ACT
     Ala Pro Gly Gly Ala Arg Arg Leu Thr Gly↓Asp Pro Arg Ile Cys
     GCA CCC GGC GGT GCT CGC CGT TTA ACG GGT GAT CCC AGG ATA TGT
     Phe Asn His Gln Ser Ser Gln Pro Gln Thr Thr Lys Thr Gln Ser
     TTT AAC CAT CAG TCA TCG CAA CCG CAA ACC ACT AAA ACT TGT TCA
     Pro Gly Glu Ser Ser Cys Tyr Asn Lys Gln Trp Ser Asp Phe Arg
     CCT GGG GAG AGC TCT TGC TAT AAC AAG CAA TGG AGC GAT TTC CGT
     Gly Thr Ile Ile Glu Arg Gly Cys Gly Cys Pro Thr Val Lys Pro
     GGA ACT ATA ATT GAA AGG GGA TGT GGT TGC CCC ACA GTG AAG CCC
     Gly Ile Lys Leu Ser Cys Cys Glu Ser Glu Val Cys Asn Asn↓Asp
     GGT ATT AAA CTC AGT TGT TGC GAA TCA GAG GTC TGC AAC AAT GAT
     Gln Thr
```

CAG ACT-fragment codant pour au moins les 422 aminoacides restants C-terminaux de la phosphatase alcaline, et est dénommée ea/phoA28.

La première flèche correspond au début de l'insert contenant l'érabutoxine a ; la deuxième flèche correspond à la fin dudit insert.

La séquence complète du gène de structure de la phosphatase alcaline (gène phoA) est décrite dans GENE, 1986, 44, 121-125.

Selon une autre disposition avantageuse de ce mode de réalisation, la séquence nucléique hybride conforme à l'invention comprend successivement la séquence leader du gène de structure de la phosphatase alcaline, un fragment codant pour les 6 aminoacides N-terminaux de la phosphatase alcaline mature, une séquence d'acide nucléique codant pour une protéine P2 ou un fragment de celle-ci, puis un fragment codant pour les 444 aminoacides restants C-terminaux de la phosphatase alcaline, laquelle séquence comprend en outre, notamment pour la mise en phase de lecture de l'ensemble de ces fragments, en aval et/ou en amont du fragment codant pour la protéine P2, un fragment d'acide nucléique approprié, laquelle séquence hybride, en phase de lecture, code pour une protéine hybride présentant à la fois les propriétés de la phosphatase alcaline et certaines des propriétés de la protéine différente de l'enzyme, notamment celle d'interagir spécifiquement avec un antigène, un anticorps ou un récepteur.

Une séquence d'acide nucléique hybride conforme à l'invention, dans laquelle la séquence codant pour la protéine P2 est insérée après le triplet codant pour le 6ème aminoacide de la phosphatase alcaline mature est notamment préparée grâce à l'insertion d'au moins un site de restriction unique dans la séquence codant pour la phosphatase alcaline, déphasant le cadre de lecture du gène de la phoA et empêchant l'expression de la phosphatase alcaline, alors que l'insertion de la protéine P2 au niveau dudit site, permet, dans les conditions de l'invention, le rephasage du gène de la phosphatase alcaline et l'expression de cette dernière.

Selon une modalité avantageuse de cette disposition, ladite séquence d'ADN hybride comprend successivement la séquence leader du gène de structure de la phosphatase alcaline, la séquence codant pour les 6 aminoacides N-terminaux de la phosphatase alcaline, la séquence d'acide nucléique codant pour l'angiotensine I, un fragment comprenant la séquence AGG G et qui permet de rephaser le gène de la phosphatase alcaline, puis un fragment codant pour, au moins, les 444 aminoacides restants C-terminaux de la phosphatase alcaline.

Ladite séquence hybride comprend la séquence n° 2 ci-après :

```
-21
GTG AAA CAA AGC ACT ATT GCA CTG GCA CTC TTA CCG TTA CTG
Met lys gln ser thr ile ala leu ala leu leu pro leu leu

                                -1  +1                  +6
TTT ACC CCT GTG ACA AAA GCC CGG ACA CCA GAA ATG ccc GAT
phe thr pro val thr lys ala arg thr pro glu met pro asp

                                            +7
CGT GTC TAT ATT CAC CCG TTT CAC CTT Agg gTT CTG GAA AAC
arg val tyr ile his pro phe his leu arg val leu glu asn

CGG GCT GCT CAG GGC GAT ATT ACT GCA CCC GGC GGT GCT CGC
arg ala ala gln gly asp ile thr ala pro gly gly ala arg

CGT TTA ACG ...
arg leu thr ...
.
.
.           +450
... CTG AAA TAA
... leu lys stop
```

Cette séquence n° 2 est dénommée Angio/phoA6.

Dans cette séquence n° 2, la numérotation négative correspond aux acides aminés du peptide signal de la phosphatase alcaline et la numérotation positive correspond aux acides aminés de la phosphatase alcaline mature. La séquence soulignée est celle de l'angiotensine I. L'arginine précédant la valine +7 a été introduite par les nécessités du clonage et la remise en phase du gène de la phoA. Les nucléotides, correspondant au site de restriction unique défini ci-dessus introduit dans le gène de la phosphatase, sont indiqués en lettres minuscules ; il s'agit dans le cas présent d'un site SmaI (ccc.....ggg). Cela permet de mettre en évidence le déphasage du gène phoA entraîné par la présence dudit site et le rephasage apporté par l'introduction de la séquence de l'angiotensine. La portion de séquence de la phosphatase non représentée (pointillés) correspond à celle publiée par CHANG et al. (CHANG, C.N., W.-J. KUANG, and E.Y. CHEN, 1986, Gene, 44:121-125).

Selon une autre modalité avantageuse de cette disposition, ladite séquence d'ADN hybride comprend successivement la séquence leader du gène de structure de la phosphatase alcaline, la séquence codant pour les 6 aminoacides N-terminaux de la phosphatase alcaline, un fragment comprenant la séquence GAT CCC, la séquence d'acide nucléique codant pour l'érabutoxine a, un fragment comprenant la séquence GAT C, lequel fragment permet de rephaser le gène de la phosphatase alcaline, puis un fragment codant pour, au moins, les 444 aminoacides restants C-terminaux de la phosphatase alcaline.

Ladite séquence hybride comprend la séquence n° 3 ci-après :

```
-21
GTG AAA CAA AGC ACT ATT GCA CTG GCA CTC TTA CCG TTA CTG
Met lys gln ser thr ile ala leu ala leu leu pro leu leu

                              -1  +1                      +6
TTT ACC CCT GTG ACA AAA GCC CGG ACA CCA GAA ATG ccc GAT
phe thr pro val thr lys ala arg thr pro glu met pro asp

CCC AGG ATA TGT TTT AAC CAT CAG TCA TCG CAA CCG CAA ACC
pro arg ile cys phe asn his gln ser ser gln pro gln thr

ACT AAA ACT TGT TCA CCT GGG GAG AGC TCT TGC TAT AAC AAG
thr lys thr cys ser pro gly glu ser ser cys tyr asn lys

CAA TGG AGC GAT TTC CGT GGA ACT ATA ATT GAA AGG GGA TGT
gln trp ser asp phe arg gly thr ile ile glu arg gly cys

GGT TGC CCC ACA GTG AAG CCC GGT ATT AAA CTC AGT TGT TGC
gly cys pro thr val lys pro gly ile lys leu ser cys cys

                                      +7
GAA TCA GAG GTC TGC AAC AAT GAT Cgg gTT CTG GAA AAC CGG
glu ser glu val cys asn asn asp arg val leu glu asn arg

GCT GCT CAG GGC GAT ATT ACT GCA CCC GGC GGT GCT CGC CGT
ala ala gln gly asp ile thr ala pro gly gly ala arg arg

TTA ACG GGT GAT CAG ACT GCC ...
leu thr gly asp gln thr ala ...
.

.
.        +450
... CTG AAA TAA
... leu lys stop
```

Cette séquence n° 3 est dénommée ea/phoA6.

Dans cette séquence n° 3, la numérotation négative correspond aux acides aminés du peptide signal de la phosphatase alcaline et la numérotation positive correspond aux acides aminés de la phosphatase alcaline mature. La séquence soulignée est celle de l'érabutoxine a. Les acides aminés Asp-Pro et Asp-Arg de part et d'autre de la séquence de l'érabutoxine ont été introduits par les nécessités du clonage et la remise en phase du gène de la phoA. Les nucléotides correspondant au site de restriction Smal introduit dans le gène de la phosphatase sont indiqués en lettres minuscules (ccc.....ggg). Cela permet de mettre en évidence le déphasage du gène phoA, entraîné par la présence dudit site et le rephasage apporté par l'introduction de la séquence de l'érabutoxine. La portion de séquence de la phosphatase non représentée (pointillés) correspond à celle publiée par CHANG et al. (CHANG, C.N., W.-J. KUANG, and E.Y. CHEN, 1986, Gene 44:121-125).

La présente invention a également pour objet une protéine, constituée par une séquence hybride comprenant un fragment de protéine étrangère inséré dans une protéine support comprenant un fragment d'une protéine P1 exportée ou sécrétée par un microorganisme notamment choisi dans le groupe qui comprend les bactéries et les levures, et un fragment fonctionnel d'une enzyme appropriée, laquelle séquence est caractérisée en ce qu'une protéine P2 appropriée est insérée entre ledit fragment de protéine P1 exportée ou sécrétée par un microorganisme et ledit fragment fonctionnel d'une enzyme appropriée, ladite protéine hybride possédant à la fois les propriétés de l'enzyme et certaines propriétés de la protéine P2, notamment celle d'interagir spécifiquement avec un anticorps, un antigène ou un récepteur.

Selon un mode de réalisation avantageux de ladite protéine hybride, la protéine P1 est différente de l'enzyme.

Selon un autre mode de réalisation avantageux de ladite protéine hybride, la protéine P1 est identique à l'enzyme.

Selon une disposition avantageuse de ce mode de réalisation, ladite protéine hybride comprend une enzyme exportée ou sécrétée par un microorganisme notamment choisi dans le groupe qui comprend les bactéries et les levures, dans laquelle est insérée une protéine P2 appropriée.

Selon une autre disposition avantageuse de ce mode de réalisation, ladite protéine hybride comprend successivement un fragment d'une enzyme exportée ou sécrétée par un microorganisme notamment choisi dans le groupe qui comprend les bactéries et les levures, une protéine P2 appropriée, puis la séquence mature complète de ladite enzyme.

Selon un autre mode de réalisation avantageux de la protéine hybride conforme à l'invention, elle contient une protéine P2 notamment choisie dans le groupe qui comprend les hormones peptidiques, les fragments simple chaîne analogues aux fragments variables des immunoglobulines et les toxines, insérée au sein d'une enzyme choisie dans le groupe qui comprend notamment la phosphatase alcaline, la phosphatase acide, la glucose phosphatase acide, la phosphodiestérase cyclique et la β-lactamase.

Selon une disposition de ce mode de réalisation, ladite protéine hybride comprend avantageusement une neurotoxine insérée dans la phosphatase alcaline.

Selon une modalité avantageuse de cette disposition, la neurotoxine est l'érabutoxine a (ea), laquelle protéine hybride présente la séquence déduite selon les formules I ou III ci-dessus.

Selon une autre disposition de ce mode de réalisation, ladite protéine hybride comprend avantageusement l'angiotensine I insérée dans la phosphatase alcaline et présente la séquence déduite selon la formule II ci-dessus.

La présente invention a notamment l'avantage d'exprimer, de manière inattendue, à la fois une enzyme et une protéine fonctionnelle P2, qu'elle soit insérée au sein de l'enzyme ou prise en sandwich entre une protéine P1 et ladite enzyme et de présenter une grande stabilité. Elle a également l'avantage d'exprimer une protéine fonctionnelle de petite taille qui, jusqu'à présent, même si elle pouvait être couplée chimiquement avec une enzyme, ne pouvait pas trouver application comme réactif et ne pouvait, en conséquence pas être utilisée dans des dosages de type EIA ou ELISA, mais uniquement dans des dosages de type RIA, beaucoup plus lourds à mettre en oeuvre.

La présente invention a également pour objet une famille de vecteurs d'expression et/ou de clonage d'une protéine hybride conforme à l'invention, caractérisée en ce que chaque vecteur inclut un système d'expression comprenant :

- un promoteur approprié,
- un site de fixation aux ribosomes,
- une séquence d'acide nucléique comportant la séquence leader du gène de structure d'une protéine P1, une séquence codant pour un fragment $NH_2$-terminal de ladite protéine P1 mature, une séquence codant pour une protéine P2 insérée au niveau d'un site de restriction naturel et la séquence codant pour une enzyme extracytoplasmique mature ou un fragment de celle-ci, laquelle séquence d'acide nucléique correspond à une séquence hybride conforme à l'invention, et
- un terminateur de transcription,

lequel système d'expression est inséré dans une structure génétique appropriée, notamment choisie dans le groupe qui comprend des plasmides, des phages, des cosmides ou des chromosomes appropriés.

Selon un mode de réalisation avantageux dudit vecteur, la structure génétique est un plasmide et la protéine P1 est identique à l'enzyme.

Selon une disposition avantageuse de ce vecteur, on obtient, lorsque P2 est avantageusement l'érabutoxine a, un plasmide présentant les propriétés suivantes :

- il comprend 6,1 kb ;
- il est obtenu par ligation du plasmide pJC2431 portant le gène de structure de la phosphatase alcaline et le gène de résistance à l'ampicilline ($Ap^R$), -ledit plasmide étant linéarisé au site Bcl I, au niveau du codon 28 dudit gène de structure de la phosphatase alcaline, -avec le fragment Sau 3AI-Sau 3AI de 192 paires de bases codant pour l'érabutoxine a.

Ce plasmide a été dénommé pEP1726 par les Inventeurs.

Le plasmide pJC2431 est décrit dans l'article de JC. LAZZARONI et al., paru dans J. Bacteriol., 1985, 164, 1376-1380.

La présente invention a également pour objet une autre famille de vecteurs d'expression et/ou de clonage d'une protéine hybride conforme à l'invention, caractérisée en ce que chaque vecteur inclut un système d'expression comprenant :

- un promoteur approprié,
- un site de fixation aux ribosomes,
- une séquence d'acide nucléique comportant la séquence leader du gène de structure d'une protéine P1, une séquence codant pour un fragment $NH_2$-terminal de ladite protéine P1 mature, la séquence codant pour une enzyme extracytoplasmique mature ou un fragment de celle-ci et un ou plusieurs sites de restriction uniques, aptes à recevoir une séquence codant pour une protéine P2, situés à la jonction entre le fragment de séquence codant pour la protéine P1 et la séquence codant pour l'enzyme, et

- un terminateur de transcription,

lequel système d'expression est inséré dans une structure génétique appropriée, notamment choisi dans le groupe qui comprend des plasmides, des phages, des cosmides ou des chromosomes appropriés.

Selon un mode de réalisation avantageux dudit vecteur, au moins l'un des sites de restriction uniques introduit entraîne un déphasage du gène de l'enzyme, le rephasage dudit gène étant assuré par l'introduction de la séquence de la protéine P2.

Selon une disposition avantageuse de ce mode de réalisation, on obtient un plasmide présentant les propriétés suivantes :

- il comprend environ 5,9 kb, et
- il est obtenu par mutagénèse dirigée du gène phoA porté par le plasmide pJC2431 de manière à introduire un site de restriction unique SmaI, correspondant à la position +6 de la protéine mature.

Un tel plasmide a été dénommé pLIPI par les Inventeurs et est dit non chargé.

Lorsque la séquence codant pour la protéine P2 est insérée au niveau d'un site de restriction naturellement présent dans le gène de structure de la phoA, ou lorsque la séquence codant pour la protéine P2 est insérée au niveau d'un site de restriction unique, préalablement introduit dans le gène de structure de la phoA, de tels vecteurs contiennent des séquences hybrides conformes à l'invention et expriment directement la protéine hybride quand ils sont présents dans un microorganisme approprié ; de tels vecteurs sont dits chargés, alors que le vecteur dénommé pLIP1, apte à recevoir une protéine P2 est, quant à lui, dit non chargé.

La présente invention a également pour objet un microorganisme obtenu par transformation génétique, caractérisé en ce qu'il est obtenu par modification appropriée d'une souche appropriée d'E. *Coli,* par un vecteur conforme à l'invention et notamment par transformation, lorsque ledit vecteur est un plasmide.

Selon un mode de réalisation de l'invention, ledit microorganisme est avantageusement une souche CC118 d'E. *Coli* transformée par le plasmide pEP1726.

Une telle souche transformée par ledit plasmide a été déposée en date du 2 juin 1989 sous le numéro I-862 auprès de la Collection Nationale des Cultures de Microorganismes tenue par l'Institut Pasteur.

Ladite souche est dénommée SEP 1726 par les Inventeurs.

Selon un autre mode de réalisation de l'invention, ledit microorganisme est avantageusement une souche CC118 d'E. *Coli* transformée par le plasmide pLIP1.

Une telle souche transformée par ledit plasmide a été déposée en date du 7 juin 1990 sous le numéro I-954 auprès de la Collection Nationale des Cultures de Microorganismes tenue par l'Institut Pasteur.

Un tel vecteur, non chargé, a l'avantage de permettre l'insertion de n'importe quelle séquence codant pour une protéine, au niveau de ce site de restriction unique.

Cette modification du gène de structure de la phosphatase alcaline introduit en outre un déphasage du cadre de lecture de la séquence nucléotidique située en aval, laquelle séquence est caractéristique de la partie fonctionnelle de la phoA.

Ce vecteur non-chargé, qui correspond au plasmide pLIP1 défini ci-dessus, n'exprime donc pas l'activité phosphatase alcaline.

La formule IV ci-après présente un alignement des séquences nucléotidiques et protéiques correspondant au gène phoA natif (A), au gène phoA modifié porté par le vecteur pLIP1 non chargé (B), et au gène phoA hybride porté par le vecteur chargé (C).

A. Phosphatase native :

```
          -
    CGG ACA CCA GAA ATG CCT GTT CTG GAA AAC CGG GCT GCT CAG
    arg thr pro glu met pro val leu glu asn arg ala ala gln
    +1              +6  +7

    ..... CTG AAA TAA
    ..... leu lys stop
          +450
```

B. Introduction du site SmaI (vecteur pLIP1 non chargé) :

```
CGG ACA CCA GAA ATG ccc ggg TTC TGG AAA ACC GGG CTG CTC
arg thr pro glu met pro gly phe trp lys thr gly leu leu
+1                  +6

..... TTT TGC TGA
..... phe cys stop
          +49
```

C. Insertion et rephasage permettant d'obtenir un hybride de la phosphatase (vecteur chargé).

```
                         insert
CGG ACA CCA GAA ATG ccc 3n +1  gg gTT CTG GAA AAC CGG GCT
arg thr pro glu met pro xxx.x  xx val leu glu asn arg ala
+1                  +6          +7

GCT ... CTG AAA TAA
ala ... leu lys stop
          +450                                      (IV)
```

Les nucléotides provenant du gène phoA natif sont indiqués en lettres majuscules. La numérotation correspond aux acides aminés de la phosphatase alcaline mature. Les pointillés correspondent aux portions de séquences non représentées. Les nucléotides correspondant au site de restriction Smal introduit dans le gène phoA sont indiqués en lettres minuscules (cccggg) (B). Le déphasage du cadre de lecture ainsi introduit entraîne la synthèse d'une protéine aberrante de 49 acides aminés (B). L'introduction au site Smal, d'une séquence de 3n+1 paires de bases (insert) ne contenant pas de codon de terminaison, permet de rephaser le gène phoA et d'obtenir une protéine hybride (C). De plus, si la séquence 3n+1 dans son orientation inverse renferme un codon de terminaison, seule l'introduction de cette séquence dans l'orientation souhaitée permettra d'obtenir une protéine présentant une activité phosphatase.

Conformément à l'invention, le promoteur est choisi dans le groupe qui comprend le promoteur du gène phoA et tout promoteur plus fort que le promoteur du gène phoA.

Le promoteur du gène phoA, notamment, est un promoteur inductible qui est activé lorsque la concentration en phosphate dans le milieu de culture des bactéries devient très faible.

Un tel promoteur a notamment l'avantage de permettre la production des protéines hybrides, dans un milieu de culture pauvre en phosphate. L'appauvrissement en phosphate du milieu au cours de la culture des bactéries permet une induction de la synthèse des protéines hybrides, après que la masse bactérienne ait augmenté. L'avantage d'un tel système inductible est de réduire les risques de protéolyse en cours de production, dans la mesure où les protéines extraites sont synthétisées en fin de culture. L'homogénéité de l'hybride s'en trouve augmentée.

La présente invention a également pour objet un processus d'expression d'une protéine hybride conforme à l'invention, caractérisé en ce qu'il met en oeuvre un vecteur d'expression tel que défini ci-dessus dans un microorganisme conforme à l'invention.

Deux propriétés de la protéine hybride conforme à l'invention doivent être soulignées :

(1) l'extrême simplicité de sa production et l'absence d'étapes longues et difficiles de purification ; et
(2) sa stabilité au cours de la production par la bactérie, pendant le stockage dans différentes conditions, et durant le test enzymatique, puisque l'activité enzymatique persiste pendant plus de 24 heures en présence du substrat.

La présente invention a également pour objet un réactif de diagnostic, caractérisé en ce qu'il est constitué par une protéine hybride conforme à l'invention.

Un tel réactif trouve notamment application dans des dosages immunoenzymologiques, dans la détection de récepteurs ou en tant que marqueurs histochimiques.

La présente invention a également pour objet un procédé de dosage immunoenzymologique de protéines, caractérisé en ce qu'il consiste à détecter des protéines présentes dans un fluide biologique en mettant en présence ledit fluide biologique avec le réactif de diagnostic conforme à l'invention et en ce que la présence dudit réactif sous forme de complexe ou sous forme libre est révélée par une réaction colorimétrique appropriée.

Selon un mode de mise en oeuvre avantageux dudit procédé, lesdites protéines sont des antigènes.

Selon un autre mode de mise en oeuvre avantageux dudit procédé, lesdites protéines sont des anticorps.

La présente invention a, de plus, pour objet une trousse prête à l'emploi pour la mise en oeuvre dudit dosage immunoenzymologique, caractérisée en ce qu'elle comprend outre les quantités appropriées de tampons et réactifs utiles pour la mise en oeuvre dudit dosage, des quantités appropriées du réactif conforme à l'invention.

Le réactif conforme à l'invention présente l'avantage d'être directement utilisable alors que habituellement, dans le cadre d'un dosage EIA ou ELISA, l'antigène ou l'anticorps est lié de façon covalente à une enzyme, le couplage étant réalisé par voie chimique. Pour être efficace, un tel couplage doit être réalisé à partir de deux composants hautement purifiés.

La présente invention a également pour objet un procédé de criblage de banques d'ADNc ou d'acide nucléique génomique ou de sélection de clones recombinants, caractérisé en ce que l'on révèle la présence éventuelle d'une enzyme exportée ou sécrétée à partir de clones de bactéries ou de cellules eucaryotes ayant éventuellement intégrées un plasmide, un phage, un cosmide ou un chromosome recombinant recherché comportant une séquence nucléique hybride conforme à l'invention par une réaction colorimétrique ou une sélection sur un milieu de culture approprié.

Selon un mode de mise en oeuvre avantageux dudit procédé, lorsque l'enzyme est la phosphatase alcaline, on révèle la présence de l'enzyme exportée ou sécrétée à l'aide d'un substrat approprié de ladite enzyme.

Selon un autre mode de mise en oeuvre avantageux dudit procédé, lorsque l'enzyme est la β-lactamase, on révèle la présence de l'enzyme exportée ou sécrétée à l'aide d'un milieu contenant de l'ampicilline.

En effet, un système d'expression comportant en aval de la séquence de la protéine P2 insérée, le gène spécifiant la β-lactamase, permet de cloner efficacement et exclusivement des phases ouvertes de lecture. Ces constructions conduisent à la synthèse de protéines hybrides comportant les polypeptides recherchés suivis de la β-lactamase fonctionnelle. Les bactéries exprimant de telles protéines sont alors sélectionnées par leur résistance à l'ampicilline.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

## Exemple 1 : Procédé de production du plasmide pEP1726.

- préparation du fragment Sau 3A I-Sau 3A I codant pour l'érabutoxine a : le fragment de restriction Sau 3A I-Sau 3A I de 192 paires de bases codant pour l'érabutoxine sans séquence signal, a été préparé à partir de la forme réplicative du phage M13mp19 (-2 GAT, -1 CCC, +63 GAT)ea contenant le gène Ea portant des mutations au niveau de 3 codons, ces modifications entraînant la formation de deux nouveaux sites de restriction Sau 3 AI dans le gène : -2 TAC (Tyr) devient GAT (Asp) ; -1 ACC (Thr) devient CCC (Pro) ; +63 TAG (AMB) devient GAT (Asp) (voir formule I) ; ce fragment est mis en présence du vecteur pJC2431 ouvert par coupure partielle avec Bcl I et d'ADN ligase. Les ligations ont été effectuées dans le tampon 50 mM Tris-HC1 pH 7,4 ; 10 mM $MgCl_2$ 10 mM DTT ; 0,5 mM ATP ; 100 $\mu$g/ml BSA ; avec 1 U d'ADN ligase du phage T4 et un rapport de concentrations vecteur/insert de 1/10. La déphosphorylation du vecteur a été réalisée d'après Maniatis et al.

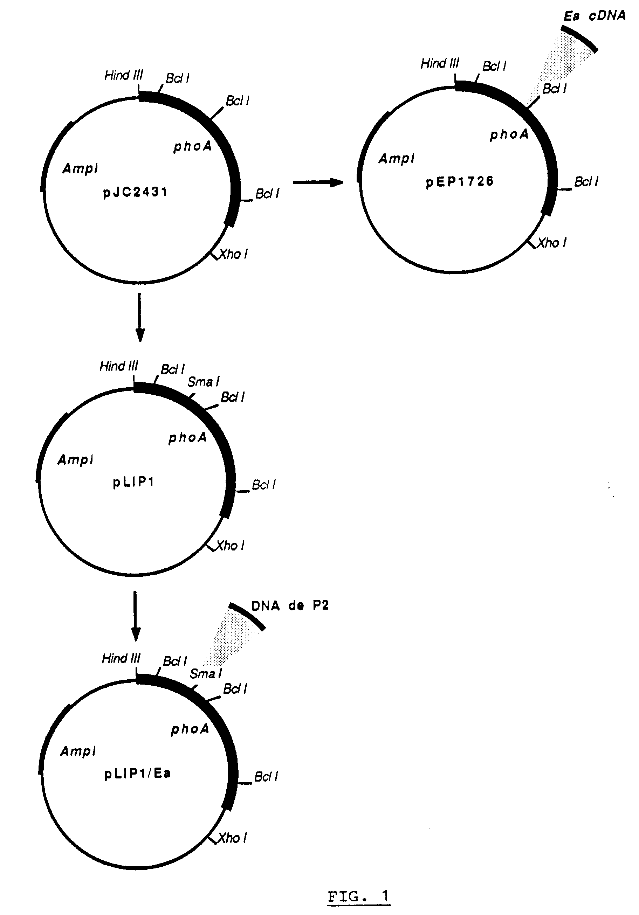

- La figure 1 représente le plasmide pJC2431 portant le gène de structure de la phosphatase alcaline sous le contrôle de son propre promoteur (Lazzaroni et al., 1985). Ce plasmide présente trois sites de restrictions Bcl I : Bcl I[1] situé en amont du promoteur de phoA, Bcl I[2] et Bcl I[3] situés respectivement au niveau des codons +28 et +363 du gène phoA ; l'insertion de l'ea dans le site Bcl I[2] permet d'obtenir le plasmide pEP1726 présentant un intérêt pour la production d'une protéine hybride conforme à l'invention.

L'enzyme Bcl I ne pouvant couper l'ADN au niveau des sites de restriction méthylés par la dam-méthylase, une souche dam- GM2163 est transformée par pJC2431, afin de préparer du plasmide non méthylé. On procède ensuite à une digestion partielle de pJC2431 par Bcl I, et à une déphosphorylation. L'ADN correspondant au plasmide ouvert (une seule coupure par Bcl I sur les trois possibles) est ensuite isolé sur un gel d'agarose à bas point de fusion suivant le protocole de Maniatis et al. (1982).

## Exemple 2 : Expression de la protéine hybride ea/phoA28. (insertion au niveau aa28 de la phosphatase alcaline).

La souche CC118 (ΔphoA20) a été transformée avec le mélange de ligation décrit à l'exemple 1. Les transformants Ap[r] (résistants à l'ampicilline) obtenus ont été purifiés sur milieu inducteur de la phosphatase alcaline contenant du XP et de l'ampicilline.

Le milieu inducteur est le TES, (solution de : $(NH_4)_2SO_4$ 2 g/l $FeSO_4,7H_2O$ 0,5 mg/l ; KCl 75 mg/l ; triéthanolamine 7,5 g/l ; ajustée à pH 7,5). L'addition du substrat chromogène 5-bromo-4-chloro-3-indolylphosphate (XP 0,4 g/l) à ce milieu permet de révéler l'activité phosphatase alcaline sur boîte de pétri, le XP conférant une coloration bleu aux colonies qui l'hydrolysent.

Trois types de transformants ont été distingués suivant leur capacité à hydrolyser le XP : des clones blancs, des clones bleus et enfin des clones bleus très foncés. Ces deux dernières catégories présentent donc une activité phosphatase alcaline due au plasmide qu'elles contiennent.

L'analyse des fragments de restriction de l'ADN plasmidique de ces différents transformants a permis de déterminer le site et l'orientation de l'insertion du gène ea dans chaque cas.

Les clones bleu foncé correspondent à des insertions d'un ou deux fragments Sau 3A I-Sau 3A I au niveau du site Bcl I[1] c'est-à-dire en amont du gène phoA et de son promoteur. L'activité phosphatase alcaline de ces clones est identique à celle de la souche CC118 (pJC2431).

Les clones bleus moins prononcés correspondent à une insertion en phase d'une copie du gène ea au niveau du site Bcl I[2] (pEP1726).

Les clones blancs proviennent de l'insertion du gêne ea au niveau du site Bcl I[2] mais dans l'orientation inverse de celle du gène phoA.

Le protocole utilisé pour l'extraction des protéines périplasmiques par choc osmotique est celui décrit par Dvorak et al. (1967).

### Exemple 3 : Procédé d'obtention du plasmide pL1P1 (vecteur d'expression non chargé) et du plasmide pLIP1/P2 (vecteur d'expression chargé).

1. Introduction d'un site de clonage dans le gène phoA.

Le gène phoA (phosphatase alcaline de *E. Coli*) porté par le plasmide pJC2431 a été modifié par mutagénèse dirigée afin d'introduire un site de restriction unique Smal après le codon correspondant au sixième acide aminé de la protéine mature, a été choisi pour deux raisons :

- la région N-terminale de la phosphatase peut être modifiée sans altération majeure de son activité enzymatique,
- la présence des premiers acides aminés N-terminaux est nécessaire pour permettre le clivage du peptide signal.

Ce site a été introduit de manière à entraîner un décalage du cadre de lecture du gène phoA. En conséquence, ce gène modifié est incapable d'exprimer une protéine fonctionnelle.

2. Introduction de séquences d'ADN exogènes dans le gène phoA.

Les fragments d'ADN destinés à être introduits dans le gène phoA au site Smal sont obtenus par synthèse nucléotidique ou proviennent de séquences géniques clonées. Ils sont préparés de manière à répondre aux exigences suivantes :

(1) leurs extrémités 5' et 3' doivent être franches, le site Smal générant des extrémités franches.

(2) leur introduction dans l'orientation souhaitée doit permettre le rephasage de la séquence du gène phoA en aval du site d'insertion et doit permettre la synthèse d'une protéine hybride présentant une activité phosphatase.

(3) leur séquence dans l'orientation inverse de celle souhaitée doit présenter un codon de terminaison en phase avec le cadre de lecture. Ceci doit entraîner une absence de fonction du gène hybride obtenu en cas d'insertion dans cette orientation.

Les constructions géniques sont obtenues par les techniques classiques de génie génétique.

Seuls les vecteurs ayant inséré les séquences exogènes dans le bon sens ont une phase ouverte de lecture permettant l'expression d'une protéine hybride constituée du polypeptide codé par la séquence insérée et de la phosphatase alcaline.

3. Sélection des gènes hybrides.

Après ligation du fragment d'ADN souhaité dans le vecteur pLIP1, les plasmides recombinants sont utilisés pour transformer la souche bactérienne CC118, mutant de E. *Coli* n'exprimant pas la phosphatase alcaline. Seuls les clones bactériens renfermant la construction génique attendue seront susceptibles de récupérer une activité phosphatase. Cette activité peut être visualisée en cultivant les bactéries sur un milieu de sélection approprié contenant un substrat chromogène de la phosphatase. Les clones positifs apparaissent en bleu.

Ainsi, l'apparition de la coloration bleue, indiquant que la bactérie exprime une protéine présentant une activité phosphatase, signifie :

- que le fragment d'ADN exogène a été inséré correctement, dans la bonne orientation, permettant un rephasage de la séquence codante de la phosphatase,
- que la protéine hybride, produit du gène recombinant, est correctement exportée dans le périplasme de la bactérie,

- que la protéine hybride réalise des dimères afin de former l'homodimére correspondant à la forme active de la phosphatase,
- et donc que l'insertion de la séquence protéique exogène dans la phosphatase n'altère pas de façon importante son activité enzymatique.

**Exemple 4 : Expression des protéines hybrides ea/phoA6 et Angio/phoA6 :**

1. Introduction d'une protéine P2 dans la phosphatase alcaline :
Elle est réalisée entre les acides aminés 6 et 7 de la phosphatase mature. Lesdites protéines hybrides sont constituées :

- des 21 acides aminés du peptide signal de la phosphatase. Ce peptide permet l'exportation de la protéine vers l'espace périplasmique de la bactérie. Cette localisation est essentielle pour l'acquisition de l'activité phosphatase et pour une extraction commode de la protéine hybride.
- des 6 acides aminés N-terminaux de la phosphatase. Leur présence permet le clivage du peptide signal pendant le passage dans le périplasme.
- de la séquence des acides aminés de la protéine exogène insérée dans la phosphatase. Les impératifs de clonage peuvent éventuellement introduire aux extrémités de cette séquence un ou quelques acides aminés supplémentaires.
- des 444 derniers acides aminés de la phosphatase.

2. Production des protéines hybrides :
Les bactéries sont cultivées en milieu liquide pauvre en phosphates. Lorsque le phosphate est épuisé, le promoteur phosphatase est activé et entraîne la transcription du gène hybride. L'intérêt de ce phénomène d'induction est de permettre une synthèse rapide des protéines hybrides en fin de culture (4 à 5 heures de culture) et donc de limiter les risques de dégradation protéolytique. Les protéines du périplasme bactérien sont extraites par choc osmotique. L'utilisation des hybrides protéiques (comme réactifs par exemple) ne nécessite pas de purification supplémentaire.
3. Hybride toxine/phosphatase (ea/phoA6) .

- Construction génique :
Un fragment de 196 paires de bases comprenant la séquence codante de l'érabutoxine a, flanquée aux extrémités 5' et 3' d'un site Sau3AI, comme précisé ci-dessus à l'exemple 1 a été réparé à la Klenow et introduit au site SmaI du plasmide pLIP1. La présence d'un codon de terminaison en phase, dans le fragment en orientation inverse, a permis une sélection visuelle des clones recombinants recherchés selon le principe précédemment décrit à l'exemple 2. Le plasmide ainsi obtenu a été dénommé pLIP1/Ea. La chaîne polypeptidique attendue (ea/phoA6) comprend successivement : le peptide signal de la phosphatase, les 6 premiers acides aminés de la phosphatase, deux acides aminés supplémentaires introduits par les nécessités du clonage (Asp, Pro), les 62 acides aminés de l'érabutoxine a, deux acides aminés supplémentaires introduits par les nécessités du clonage (Asp, Arg), les 444 derniers acides aminés de la phosphatase. Le peptide signal est clivé lors du passage dans le périplasme.
- Caractéristiques de la protéine hybride ea/phoA6 :

La protéine hybride ea/phoA6 a été produite selon les modalités décrites précédemment.
Trois caractéristiques de l'hybride ont été étudiées :

(1) son activité enzymatique,
(2) la présence de déterminants antigéniques spécifiques de l'érabutoxine,
(3) la possibilité d'utiliser l'hybride pour effectuer un dosage de toxine libre.

Ces caractéristiques ont été comparées avec celles de l'hybride ea/phoA28 (exemple 2), produit du plasmide pEP1726, obtenu par introduction de l'érabutoxine dans la phosphatase après la position 28, sans modification préalable du gène phoA, en utilisant un site de restriction préexistant.
a. L'activité enzymatique des hybrides a été évaluée par comparaison avec celle de la phosphatase alcaline native obtenue dans les mêmes conditions de production. L'activité spécifique d'extraits périplasmiques bactériens, obtenus par choc osmotique, contenant les différentes protéines a été déterminée. La concentration en protéines de l'extrait est déterminée à partir de son absorbance à 546 nm et d'une courbe-étalon réalisée par dosage des protéines par la méthode de Bradford (Bradford, 1976). L'activité enzymatique est mesurée sur 20 µl d'extrait. Après 15 min d'incubation à 37°C en présente de p-nitro phényl phosphate (pNPP) 25 mM, la réaction est arrêtée par l'addition de 1 ml de NaOH 1M. L'absorbance de l'échantillon est lue à 410 nm (coloration jaune due à la formation de pNPOH) . L'activité enzymatique spécifique (AS), en nmoles de pNPP hydrolysé par minute par mg de protéines, est donnée par la formule :

$$AS = \frac{A410.\ 5,55}{(\text{mg protéines/ml}).2.10^{-2}}$$

L'évaluation de l'activité des hybrides est exprimée en pourcentage d'activité spécifique correspondant à la phosphatase native :

| Phosphatase native : | 100 % (A.S.) |
|---|---|
| ea/phoA6 : | 50 % |
| ea/pho28 : | 30 % |

b. la présence de déterminants antigéniques spécifiques de l'érabutoxine portés par la protéine hybride ea/phoA6 et ea/phoA28 a été étudiée par test ELISA. Ces protéines sont capables de se fixer sur l'anticorps monoclonal Mα2-3, spécifique d'un déterminant antigénique porté par la toxine native. De plus, il existe une compétition de fixation entre ea/phoA6 ou ea/phoA28 et la toxine native. Afin d'évaluer le niveau de structuration de la toxine dans les hybrides, on a comparé par test ELISA, sa reconnaissance par un sérum dirigé contre la toxine native et par un sérum dirigé contre la toxine dénaturée. Pour 60 % de l'hybride ea/phoA6, la toxine est correctement structurée, alors que cette proportion n'est que de 32 % pour l'hybride ea/phoA28.

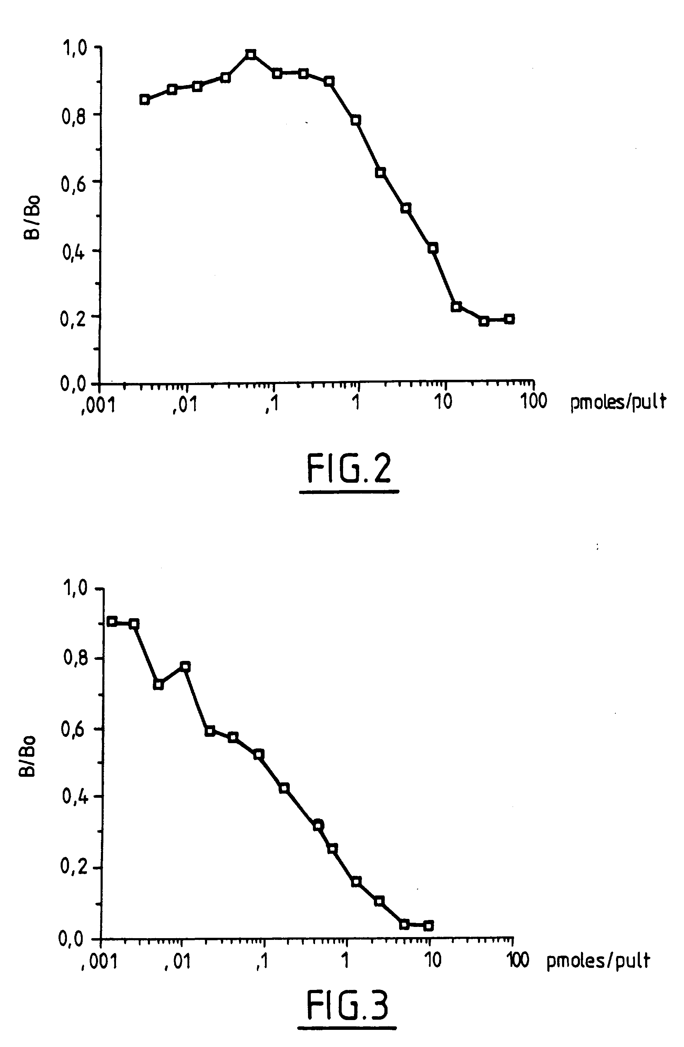

c. La figure 2 illustre un dosage de l'erabutoxine a par un test ELISA utilisant l'hybride ea/phoA6.

La compétition de fixation entre ea/PhoA6 et la toxine libre sur l'anticorps monoclonal Mα2-3 a été étudiée par test ELISA. Après adsorption de l'anticorps sur plaque ELISA, ea/phoA6 (choc osmotique dilué 10 fois) est ajoutée en présence de différentes quantités de toxine.

$$\text{Le rapport}\ \frac{B}{Bo} = \frac{\text{quantité d'hybride fixé en présence de toxine}}{\text{quantité d'hybride fixé en l'absence de toxine}}$$

est exprimé en fonction de la quantité de toxine libre. La sensibilité de ce test, évaluée par la quantité de toxine pour laquelle le rapport B/Bo=0,5, est de 2,5 pmole, soit 17 ng. Cette sensibilité est équivalente à celle d'un test RIA utilisant de la toxine tritiée.

4. Hybride angiotensine I/phosphatase alcaline (Angio/phoA6) .

- Construction génique :

Un fragment de 31 paires de bases capable de coder pour l'angiotensine I a été obtenu par hybridation de deux oligonucléotides synthétiques complémentaires. Ce fragment a été introduit au site SmaI du plasmide pLIP1. La séquence de ce fragment a été déterminée de manière à introduire un codon de terminaison en phase avec le cadre de lecture de la phosphatase lorsque son intégration se produit dans l'orientation inverse de celle souhaitée. Ainsi, après séquençage de plusieurs clones sélectionnés pour l'expression d'une activité phosphatase (clones bleus), tous correspondaient à la construction souhaitée. Le plasmide ainsi obtenu a été dénommé pLIP1/Angio. La chaîne polypeptidique attendue (Angio/phoA6) comprend successivement : le peptide signal de la phosphatase, les 6 premiers acides aminés de la phosphatase, les 10 acides aminés de l'angiotensine 1, un acide aminé supplémentaire introduit par les nécessités du clonage (Arg), les 444 derniers acides aminés de la phosphatase. Le peptide signal est clivé lors du passage dans le périplasme.

- Caractéristiques de la protéine hybride Angio/phoA6 :

La protéine hybride Angio/phoA6 a été produite selon les modalités décrites précédemment.

Trois caractéristiques de l'hybride ont été étudiées :

(1) son activité enzymatique,
(2) sa reconnaissance par un sérum dirigé contre l'angiotensine I,
(3) la possibilité d'utiliser l'hybride pour effectuer un dosage hormonal.

a. L'activité enzymatique de l'hybride Angio/phoA6 a été évaluée selon la procédure décrite précédemment pour l'hybride ea/phoA6. Cette évaluation est exprimée en pourcentage d'activité spécifique correspondant à la phosphatase native :

| Phosphatase native : | 100 % (A.S.) |
|---|---|
| Angio/phoA6 : | 93 % |

b. La reconnaissance de la protéine hybride Angio/phoA6 par un sérum dirigé contre l'angiotensine I a été étudiée par test ELISA. Un sérum de porc dirigé contre des immunoglobulines de lapin a été adsorbé sur une plaque ELISA. Un sérum de lapin dirigé contre de l'angiotensine I couplée à de la sérum albumine bovine a ensuite été fixé sur le sérum de porc. Enfin, la protéine hybride Angio/PhoA6, produite par choc osmotique, a été ajoutée au système. Sa fixation sur les anticorps dirigés contre l'angiotensine a été révélée par son activité phosphatase, en ajoutant un substrat chromogène de l'enzyme. La spécificité de cette reconnaissance est démontrée par l'existence d'une compétition de fixation entre l'hybride et de l'hormone libre.

c. La figure 3 illustre un dosage de l'angiotensine I par test ELISA utilisant l'hybride Angio/phoA6.

La compétition de fixation entre Angio/phoA6 et de l'angiotensine I libre sur un sérum de lapin dirigé contre cette hormone a été étudiée par test ELISA. Le sérum de lapin est fixé au préalable par un sérum de porc anti-immunoglobuline de lapin adsorbé sur plaque ELISA. L'hybride Angio/phoA6 (choc osmotique dilué 20 fois) est ajouté en présence de différentes quantités d'angiotensine I.

$$\text{Le rapport} : \frac{B}{Bo} = \frac{\text{quantité d'hybride fixé en présence d'hormone}}{\text{quantité d'hybride fixé en l'absence d'hormone}}$$

est exprimé en fonction de la quantité d'hormone libre. La sensibilité de ce test, évaluée par la quantité d'hormone pour laquelle le rapport B/Bo=0,5, est de 0,09 pmole, soit 0,12 ng. Cette sensibilité est équivalente à celle de tests RIA utilisés en pratique diagnostique courante (kit commercialisé par COMPAGNIE ORIS INDUSTRIE FRANCE).

**Exemple 5 : Stabilité des protéines hybrides de la phosphatase.**

1. Induction de la synthèse des hybrides de la phosphatase :

Dans le vecteur d'expression réalisé, pLIP1, le promoteur du gène phoA a été conservé pour permettre l'expression des gênes hybrides. Il s'agit d'un promoteur inductible qui est activé lorsque la concentration en phosphate dans le milieu de culture des bactéries devient très faible.

Dans les conditions choisies (voir exemple 4.2), la synthèse des protéines hybrides, mesurée par apparition de l'activité phosphatase, débute après 3 heures 30 de culture. L'extraction se fait lorsque le maximum d'accumulation des hybrides est atteint, soit après 4 heures 30 à 5 heures.

2. Stabilité des protéines hybrides :

La stabilité des protéines hybrides a été étudiée dans trois situations :

(1)     stabilité au cours de leur production,
(2)     stabilité au cours de leur utilisation,
(3)     stabilité au cours des opérations de stockage.

a. La stabilité des hybrides et de la phosphatase native a été étudiée en visualisant leur intégrité après marquage à la méthionine $^{35}$S, immunoprécipitation, et électrophorèse en gel de polyacrylamide. Les bactéries sont cultivées dans les conditions habituelles de production. Le marquage des hybrides à la méthionine $^{35}$S est réalisé au moment de l'induction de leur synthèse pendant 30 minutes. Les protéines immunoprécipitées avec un anticorps monoclonal spécifique de la phosphatase alcaline apparaissent sous forme d'une espèce moléculaire unique. Les masses moléculaires observées sont très proches des valeurs théoriques attendues : 46 000 daltons environ pour la phosphatase, 52 000 daltons environ pour ea/phoA6, et 47 000 daltons environ pour Angio/phoA6. En prolongeant la culture bactérienne de 4 heures après le marquage (chasse), aucune dégradation des protéines marquées n'est observée. Ces résultats montrent que les protéines hybrides de la phosphatase ne subissent aucune dégradation protéolytique au cours de leur production. Ceci est loin d'être le cas pour les protéines hybrides obtenues par fusion de la protéine A du staphylocoque ou de la β-galactosidase d'E. *Coli.* Dans ces systèmes, la majeure partie de la protéine hybride attendue subit une dégradation protéolytique.

b. Après 24 heures d'incubation dans les conditions de révélation du test ELISA, aucune baisse significative d'activité enzymatique des hybrides n'est observée. Ce résultat démontre la stabilité du réactif en cours d'utilisation. En conséquence, le temps de révélation d'un test ELISA peut être prolongé afin d'en augmenter la sensibilité.

c. Après 10 cycles de congélation à -180°C suivie de décongélation à +37°C, c'est-à-dire dans des conditions drastiques, 95 % de l'activité phosphatase des hybrides est conservée. Des études préliminaires ont fourni des résultats similaires concernant la lyophilisation. Il semble donc que les hybrides de la phosphatase puissent rester parfaitement stables dans des conditions habituelles de conditionnement.

**Exemple 6 : Procédé de criblage de banques d'ADNc ou génomiques, conforme à l'invention.**

Les systèmes de clonage et d'expression associés actuellement sur le marché sont intégrés dans des bactériophages ou des plasmides. Leurs caractéristiques communes essentielles sont les suivantes : ils possèdent une partie du gène *lac Z d'E. Coli* (β-galactosidase) modifié de telle sorte qu'il contienne un, ou plus souvent une série de sites uniques de restriction. L'insertion de fragments de DNA de ce site entraîne généralement la perte de l'activité β-galactosidase. Cette propriété permet de reconnaître les clones recombinants qui forment des colonies ou des plages de lyse blanches alors que les clones sans insert apparaissent bleus en présence de X-Gal. Par ailleurs, les séquences de DNA insérées peuvent être exprimées sous forme de protéines de fusion avec un fragment de la β-galactosidase. Leur détection est alors possible à l'aide d'anticorps spécifiques des protéines recherchées. La révélation s'effectue par différentes techniques : marquage à l'iode[125] ou l'utilisation de conjugués anticorps-enzymes (Molecular Cloning, 1989, 12-14). La substitution dans ces systèmes du gène *lac Z* modifié par le gène phoA modifié notamment comme décrit pour le plasmide pLIP1, conduit à l'apparition de clones bleus et blancs en présence de XP. Dans ce cas, à l'inverse des systèmes utilisant la β-galactosidase, les clones capables de transformer le substrat colorimétrique, XP en l'occurrence, sont des clones recombinants. De plus, la simple coloration d'un clone est indicative de plusieurs propriétés. Elle signifie que : i) un fragment d'ADN a été inséré dans le gène phoA, ii) ce fragment code pour une phase ouverte de lecture, iii) l'insertion a permis le rephasage du gène phoA et son expression sous forme d'une protéine hybride, iv) cette protéine hybride est exportée dans le périplasme, v) la phosphatase alcaline recombinante dimérise pour former une enzyme active qui hydrolyse ses substrats et en particulier les substrats colorimétriques. Ainsi, les informations révélées par le test colorimétrique sont beaucoup plus nombreuses que dans le cas de la β-galactosidase. L'utilisation de cette dernière enzyme permet de distinguer les clones avec insert et les clones sans insert sans prévaloir de l'existence ou des propriétés d'une protéine recombinante, alors que l'utilisation de la phosphatase alcaline permet d'identifier directement les clones capables de synthétiser une protéine hybride exportable et possédant un domaine phoA structuré et enzymatiquement actif. Par ailleurs, la capacité des protéines hybrides à être exportées vers le périplasme n'est pas sans incidence sur la structuration du polypeptide inséré dans phoA. Dans le cas de l'érabutoxine a, son exportation s'accompagne de la formation de ses ponts disulfures. Elle adopte ainsi une conformation lui permettant d'être reconnue par des anticorps spécifiques de la forme structurée de la toxine. La plupart des anticorps ont ce type de spécificité conformation dépendante et ne reconnaissent que des protéines correctement structurées. Enfin, il est possible de tirer parti de l'activité enzymatique des protéines hybrides synthétisées pour mettre en évidence très simplement une interaction avec des anticorps spécifiques de la protéine insérée. Une simple empreinte des colonies synthétisant les protéines recombinantes sur un filtre de nitrocellulose préalablement saturé par une solution d'anticorps spécifiques, suivie d'une révélation avec un réactif colorimétrique de la phosphatase, permettra de révéler les clones sécrétant une protéine hybride capable de réagir avec les anticorps.

Ce procédé peut s'avérer extrêmement performant également pour identifier les épitopes d'une protéine définie. Des fragments d'ADN codant pour cette protéine peuvent être obtenus par synthèse, par action d'enzymes de restriction sur le gène entier ou par toute autre méthode comme l'utilisation des techniques de PCR (polymerase chain reaction) par exemple et intégrés dans le gène phoA. L'étude de l'interaction des protéines recombinantes résultant de ces constructions avec des sérums ou des anticorps monoclonaux dirigés contre la protéine entière permet alors d'identifier les régions de la protéine portant les épitopes.

**Exemple 7 : Procédé de sélection de clones recombinants conforme à l'invention :**

L'utilisation du gène *bla* qui code pour la β-lactamase, une enzyme qui confère à la bactérie qui l'exprime la résistance à l'ampicilline permet de disposer d'un crible de sélection des clones recombinants qui seuls peuvent se développer en présence de l'antibiotique. Ce type de sélection permet la recherche de séquences codantes très rares.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1. Séquence hybride d'acide nucléique du type comprenant un fragment codant pour une protéine étrangère inséré dans une séquence codant pour une protéine support comprenant la séquence leader du gène de structure d'une protéine appropriée P1 exportée ou sécrétable par un microorganisme, notamment choisi dans le groupe qui comprend les bactéries et les levures, une séquence d'acide nucléique codant pour un fragment $NH_2$-terminal de ladite protéine P1 mature, un fragment codant pour au moins un fragment fonctionnel de la séquence mature d'une enzyme appropriée, laquelle séquence est caractérisée en ce qu'une séquence d'acide nucléique codant pour une protéine P2 est insérée entre ledit fragment NH$_2$-terminal de ladite protéine P1 mature et ledit fragment codant pour au moins un fragment fonctionnel de la séquence mature d'une enzyme appropriée, l'ensemble de ces fragments étant en phase de lecture et codant pour une protéine hybride présentant à la fois les propriétés de l'enzyme et certaines propriétés de ladite protéine P2, notamment celle d'interagir spécifiquement avec un anticorps, un antigène ou un récepteur.

2. Séquence hybride selon la revendication 1, caractérisée en ce que l'enzyme est choisie dans le groupe qui comprend notamment la phosphatase alcaline, la phosphatase acide, la glucose phosphatase acide, la phosphodiestérase cyclique et la β-lactamase.

3. Séquence hybride selon la revendication 1 ou la revendication 2, caractérisé en ce que le fragment de séquence codant pour la protéine P2 est notamment choisi dans le groupe qui comprend les séquences codant pour les hormones peptidiques, les séquences codant pour une toxine et les séquences codant pour un fragment simple chaîne analogue au fragment variable des immunoglobulines (immunoglobulines recombinantes).

4. Séquence hybride selon la revendication 3, caractérisée en ce que la toxine est avantageusement une neurotoxine.

5. Séquence hybride selon l'une quelconque des revendications 1 à 4, caractérisée en ce que la protéine P1 est identique à l'enzyme.

6. Séquence hybride selon l'une quelconque des revendications 1 à 4, caractérisée en ce que la protéine P1 est différente de l'enzyme.

7. Séquence hybride selon l'une quelconque des revendications 1 à 5, caractérisée en ce qu'elle est constituée par une séquence hybride comprenant successivement le fragment leader du gène de structure d'une enzyme exportée ou sécrétée par un microorganisme, notamment choisi dans le groupe qui comprend les bactéries et les levures, une séquence d'acide nucléique codant pour un fragment NH$_2$-terminal de ladite enzyme mature, une séquence d'acide nucléique codant pour une protéine P2, puis un fragment codant pour au moins un fragment fonctionnel de la séquence mature de ladite enzyme, l'ensemble de ces fragments étant en phase de lecture et codant pour une protéine hybride présentant à la fois les propriétés de l'enzyme et certaines propriétés de ladite protéine, notamment celle d'interagir spécifiquement avec un anticorps, un antigène ou un récepteur.

8. Séquence hybride selon l'une quelconque des revendications 1 à 5 ou selon la revendication 7, caractérisée en ce qu'elle comprend successivement la séquence leader du gène de structure de la phosphatase alcaline, un fragment codant pour, au plus, les 28 amino-acides N-terminaux de la phosphatase alcaline mature, une séquence d'ADN codant pour une protéine P2 ou un fragment de celle-ci, puis un fragment codant pour, au moins, les 422 aminoacides restants C-terminaux de la phosphatase alcaline, l'ensemble de ces fragments étant en phase de lecture et formant une séquence d'ADN hybride codant pour une protéine hybride présentant à la fois les propriétés de la phosphatase alcaline, en particulier son activité enzymatique et certaines des propriétés de la protéine P2, notamment celle d'interagir spécifiquement avec un antigène, un anticorps ou un récepteur.

9. Séquence hybride selon la revendication 8, caractérisée en ce qu'elle comprend successivement la séquence d'acide nucléique codant pour le peptide leader de la phosphatase alcaline, la séquence codant pour les 28 amino-acides N-terminaux de la phosphatase alcaline, la séquence codant pour l'érabutoxine a (ea) mature, et la séquence codant pour les 422 aminoacides C-terminaux de la phosphatase alcaline.

10. Séquence hybride selon la revendication 8 ou la revendication 9, caractérisée en ce que la séquence codant pour l'érabutoxine a comprend 192 paires de bases et deux sites de restriction Sau 3 AI supplémentaires.

11. Séquence hybride selon la revendication 10, caractérisée en ce qu'elle comprend la séquence n° 1 ci-après :

```
                                                      Arg Thr Pro
Fragment leader du gène de structure de la phoA-CGG ACA CCA
Glu Met Pro Val Leu Glu Asn Arg Ala Ala Gln Gly Asp Ile Thr
GAA ATG CCT GTT CTG GAA AAC CGG GCT GCT CAG GGC GAT ATT ACT
Ala Pro Gly Gly Ala Arg Arg Leu Thr Gly↓Asp Pro Arg Ile Cys
GCA CCC GGC GGT GCT CGC CGT TTA ACG GGT GAT CCC AGG ATA TGT
Phe Asn His Gln Ser Ser Gln Pro Gln Thr Thr Lys Thr Gln Ser
TTT AAC CAT CAG TCA TCG CAA CCG CAA ACC ACT AAA ACT TGT TCA
Pro Gly Glu Ser Ser Cys Tyr Asn Lys Gln Trp Ser Asp Phe Arg
CCT GGG GAG AGC TCT TGC TAT AAC AAG CAA TGG AGC GAT TTC CGT
Gly Thr Ile Ile Glu Arg Gly Cys Gly Cys Pro Thr Val Lys Pro
GGA ACT ATA ATT GAA AGG GGA TGT GGT TGC CCC ACA GTG AAG CCC

Gly Ile Lys Leu Ser Cys Cys Glu Ser Glu Val Cys Asn Asn↓Asp
GGT ATT AAA CTC AGT TGT TGC GAA TCA GAG GTC TGC AAC AAT GAT
Gln Thr
CAG ACT-fragment codant pour au moins les 422 aminoacides restants C-terminaux de la phosphatase alcaline.
```

**12.** Séquence hybride selon l'une quelconque des revendications 1 à 5 ou selon la revendication 7, caractérisée en ce qu'elle comprend successivement la séquence leader du gène de structure de la phosphatase alcaline, un fragment codant pour les 6 aminoacides N-terminaux de la phosphatase alcaline mature, une séquence d'acide nucléique codant pour une protéine P2 ou un fragment de celle-ci, puis un fragment codant pour les 444 aminoacides restants C-terminaux de la phosphatase alcaline, laquelle séquence comprend en outre, notamment pour la mise en phase de lecture de l'ensemble de ces fragments, en aval et/ou en amont du fragment codant pour la protéine P2, un fragment d'acide nucléique approprié, laquelle séquence hybride, en phase de lecture, code pour une protéine hybride présentant à la fois les propriétés de la phosphatase alcaline et certaines des propriétés de la protéine différente de l'enzyme, notamment celle d'interagir spécifiquement avec un antigène, un anticorps ou un récepteur.

**13.** Séquence hybride selon la revendication 12, caractérisée en ce qu'elle comprend successivement la séquence leader du gène de structure de la phosphatase alcaline, la séquence codant pour les 6 aminoacides N-terminaux de la phosphatase alcaline, la séquence d'acide nucléique codant pour l'angiotensine I, un fragment comprenant la séquence AGG G et qui permet de rephaser le gène de la phosphatase alcaline, puis un fragment codant pour, au moins, les 444 aminoacides restants C-terminaux de la phosphatase alcaline.

**14.** Séquence hybride selon la revendication 13, caractérisée en ce qu'elle comprend la séquence n° 2 ci-après :

```
-21
GTG AAA CAA AGC ACT ATT GCA CTG GCA CTC TTA CCG TTA CTG
Met lys gln ser thr ile ala leu ala leu leu pro leu leu

                                -1  +1                +6
TTT ACC CCT GTG ACA AAA GCC CGG ACA CCA GAA ATG ccc GAT
phe thr pro val thr lys ala arg thr pro glu met pro asp

                                                +7
CGT GTC TAT ATT CAC CCG TTT CAC CTT Agg gTT CTG GAA AAC
arg val tyr ile his pro phe his leu arg val leu glu asn

CGG GCT GCT CAG GGC GAT ATT ACT GCA CCC GGC GGT GCT CGC
```

```
arg ala ala gln gly asp ile thr ala pro gly gly ala arg

CGT TTA ACG ...
arg leu thr ...
    .
    .
    .        +450
... CTG AAA TAA
... leu lys stop
```

15. Séquence hybride selon la revendication 12, caractérisée en ce qu'elle comprend successivement la séquence leader du gène de structure de la phosphatase alcaline, la séquence codant pour les 6 aminoacides N-terminaux de la phosphatase alcaline, un fragment comprenant la séquence GAT CCC, la séquence d'acide nucléique codant pour l'érabutoxine a, un fragment comprenant la séquence GAT C, lequel fragment permet de rephaser le gène de la phosphatase alcaline, puis un fragment codant pour, au moins, les 444 aminoacides restants C-terminaux de la phosphatase alcaline.

16. Séquence selon la revendication 15 caractérisée en ce qu'elle comprend la séquence n° 3 ci-après :

```
-21
GTG AAA CAA AGC ACT ATT GCA CTG GCA CTC TTA CCG TTA CTG
Met lys gln ser thr ile ala leu ala leu leu pro leu leu

                                -1  +1              +6
TTT ACC CCT GTG ACA AAA GCC CGG ACA CCA GAA ATG ccc GAT
phe thr pro val thr lys ala arg thr pro glu met pro asp

CCC AGG ATA TGT TTT AAC CAT CAG TCA TCG CAA CCG CAA ACC
pro arg ile cys phe asn his gln ser ser gln pro gln thr

ACT AAA ACT TGT TCA CCT GGG GAG AGC TCT TGC TAT AAC AAG
thr lys thr cys ser pro gly glu ser ser cys tyr asn lys

CAA TGG AGC GAT TTC CGT GGA ACT ATA ATT GAA AGG GGA TGT
gln trp ser asp phe arg gly thr ile ile glu arg gly cys

GGT TGC CCC ACA GTG AAG CCC GGT ATT AAA CTC AGT TGT TGC
gly cys pro thr val lys pro gly ile lys leu ser cys cys

                                +7
GAA TCA GAG GTC TGC AAC AAT GAT Cgg gTT CTG GAA AAC CGG
glu ser glu val cys asn asn asp arg val leu glu asn arg


GCT GCT CAG GGC GAT ATT ACT GCA CCC GGC GGT GCT CGC CGT
ala ala gln gly asp ile thr ala pro gly gly ala arg arg

TTA ACG GGT GAT CAG ACT GCC ...
leu thr gly asp gln thr ala ...
    .
    .
    .        +450
... CTG AAA TAA
... leu lys stop
```

**17.** Protéine, constituée par une séquence hybride comprenant un fragment de protéine étrangère inséré dans une protéine support comprenant un fragment d'une protéine P1 exportée ou sécrétée par un microorganisme notamment choisi dans le groupe qui comprend les bactéries et les levures, et un fragment fonctionnel d'une enzyme appropriée, laquelle séquence est caractérisée en ce qu'une protéine P2 appropriée est insérée entre ledit fragment de protéine P1 exportée ou sécrétée par un microorganisme et ledit fragment fonctionnel d'une enzyme appropriée, ladite protéine hybride possédant à la fois les propriétés de l'enzyme et certaines propriétés de la protéine P2, notamment celle d'interagir spécifiquement avec un anticorps, un antigène ou un récepteur.

**18.** Protéine hybride selon la revendication 17, caractérisée en ce que la protéine P1 est différente de l'enzyme.

**19.** Protéine hybride selon la revendication 17, caractérisée en ce que la protéine P1 est identique à l'enzyme.

**20.** Protéine hybride selon la revendication 17 ou la revendication 19, caractérisée en ce qu'elle comprend une enzyme exportée ou sécrétée par un microorganisme notamment choisi dans le groupe qui comprend les bactéries et les levures, dans laquelle est insérée une protéine P2 appropriée.

**21.** Protéine hybride selon la revendication 17 ou la revendication 19, caractérisée en ce qu'elle comprend successivement un fragment d'une enzyme exportée ou sécrétée par un microorganisme notamment choisi dans le groupe qui comprend les bactéries et les levures, une protéine P2 appropriée, puis la séquence mature complète de ladite enzyme.

**22.** Protéine hybride selon l'une quelconque des revendications 17 à 21, caractérisée en ce qu'elle contient une protéine P2 notamment choisie dans le groupe qui comprend les hormones peptidiques, les fragments simple chaîne analogues aux domaines variables des immunoglobulines et les toxines, insérée au sein d'une enzyme choisie dans le groupe qui comprend notamment la phosphatase alcaline, la phosphatase acide, la glucose phosphatase acide, la phosphodiestérase cyclique et la β-lactamase.

**23.** Protéine hybride selon la revendication 22, caractérisée en ce qu'elle comprend avantageusement une neurotoxine insérée dans la phosphatase alcaline.

**24.** Protéine hybride selon la revendication 22 ou la revendication 23, caractérisée en ce que la neurotoxine est l'érabutoxine a (ea), laquelle protéine hybride présente la séquence déduite selon les formules I ou III selon la revendication 12 ou la revendication 16.

**25.** Protéine hybride selon la revendication 22, caractérisée en ce qu'elle comprend avantageusement l'angiotensine 1 insérée dans la phosphatase alcaline et présente la séquence déduite selon la formule II selon la revendication 14.

**26.** Famille de vecteurs d'expression et/ou de clonage d'une protéine hybride selon l'une quelconque des revendications 17 à 25, caractérisée en ce que chaque vecteur inclut un système d'expression comprenant :

- un promoteur approprié,
- un site de fixation aux ribosomes,
- une séquence d'acide nucléique comportant la séquence leader du gène de structure d'une protéine P1, une séquence codant pour un fragment $NH_2$-terminal de ladite protéine P1 mature, une séquence codant pour une protéine P2 insérée au niveau d'un site de restriction naturel et la séquence codant pour une enzyme extracytoplasmique mature ou un fragment de celle-ci, laquelle séquence d'acide nucléique correspond à une séquence hybride conforme à l'invention, et
- un terminateur de transcription,
  lequel système d'expression est inséré dans une structure génétique appropriée, notamment choisie dans le groupe qui comprend des plasmides, des phages, des cosmides ou des chromosomes appropriés.

**27.** Vecteur selon la revendication 26, caractérisé en ce que la structure génétique est un plasmide et la protéine P1 est identique à l'enzyme.

**28.** Vecteur selon la revendication 26 ou la revendication 27, caractérisé en ce que lorsque P2 est avantageusement l'érabutoxine a, on obtient un plasmide, dénommé pEP1726, présentant les propriétés suivantes :

- il comprend 6,1 kb ;

- il est obtenu par ligation du plasmide pJC2431 portant le gène de structure de la phosphatase alcaline et le gène de résistance à l'ampicilline (Ap$^R$), -ledit plasmide étant linéarisé au site Bcl I, au niveau du codon 28 dudit gène de structure de la phosphatase alcaline, -avec le fragment Sau 3AI-Sau 3AI de 192 paires de bases codant pour l'érabutoxine a.

29. Famille de vecteurs d'expression et/ou de clonage d'une protéine hybride selon l'une quelconque des revendications 17 à 25, caractérisée en ce que chaque vecteur inclut un système d'expression comprenant :

- un promoteur approprié,
- un site de fixation aux ribosomes,
- une séquence d'acide nucléique comportant la séquence leader du gène de structure d'une protéine P1, une séquence codant pour un fragment NH$_2$-terminal de ladite protéine P1 mature, la séquence codant pour une enzyme extracytoplasmique mature ou un fragment de celle-ci et un ou plusieurs sites de restriction uniques, aptes à recevoir une séquence codant pour une protéine P2, situés à la jonction entre le fragment de séquence codant pour la protéine P1 et la séquence codant pour l'enzyme, et
- un terminateur de transcription,
  lequel système d'expression est inséré dans une structure génétique appropriée, notamment choisi dans le groupe qui comprend des plasmides, des phages, des cosmides ou des chromosomes appropriés.

30. Vecteur selon la revendication 29, caractérisé en ce qu'au moins l'un des sites de restriction uniques introduit entraîne un déphasage du gène de l'enzyme, le rephasage dudit gène étant assuré par l'introduction de la séquence de la protéine P2.

31. Vecteur selon la revendication 29 ou la revendication 30, caractérisé en ce qu'on obtient un plasmide dénommé pLIP1 présentant les propriétés suivantes :

- il comprend environ 5,9 kb, et
- il est obtenu par mutagénèse dirigée du gène phoA porté par le plasmide pJC2431 de manière à introduire un site de restriction unique SmaI, correspondant à la position +6 de la protéine mature.

32. Microorganisme obtenu par transformation génétique, caractérisé en ce qu'il est obtenu par modification appropriée d'une souche appropriée d'E. *Coli* par un vecteur selon l'une quelconque des revendications 26 à 31.

33. Microorganisme selon la revendication 32, caractérisé en ce qu'il est avantageusement une souche CC118 *d'E. Coli* transformée par le vecteur pEP1726.

34. Microorganisme selon la revendication 33, dénommé SEP 1726, caractérisé en ce qu'ila été déposée en date du 2 juin 1989 sous le numéro I-862 auprès de la Collection Nationale des Cultures de Microorganismes tenue par l'Institut Pasteur.

35. Microorganisme selon la revendication 32, caractérisé en ce qu'il est avantageusement une souche CC118 *d'E. Coli* transformée par le plasmide pLIP1.

36. Microorganisme selon la revendication 35, caractérisé en ce qu'il a été déposée en date du 7 juin 1990 sous le numéro 1-954 auprès de la Collection Nationale des Cultures de Microorganismes tenue par l'Institut Pasteur.

37. Processus d'expression d'une protéine hybride selon l'une quelconque des revendications 17 à 25, caractérisé en ce qu'il met en oeuvre un vecteur d'expression selon l'une quelconque des revendications 26 à 31, dans un microorganisme selon l'une quelconque des revendications 32 à 36.

38. Réactif de diagnostic, caractérisé en ce qu'il est constitué par une protéine hybride selon l'une quelconque des revendications 17 à 25.

39. Procédé de dosage immunoenzymologique de protéines, caractérisé en ce qu'il consiste à détecter des protéines présentes dans un fluide biologique en mettant en présence ledit fluide biologique avec le réactif de diagnostic selon la revendication 38 et en ce que la présence dudit réactif sous forme de complexe ou sous forme libre est révélée par une réaction colorimétrique appropriée.

**40.** Procédé de dosage selon la revendication 39, caractérisé en ce que lesdites protéines sont des antigènes.

**41.** Procédé de dosage selon la revendication 39, caractérisé en ce que lesdites protéines sont des anticorps.

**42.** Trousse prête à l'emploi pour la mise en oeuvre d'un dosage enzymoimmunologique selon l'une quelconque des revendications 39 à 41, caractérisée en ce qu'elle comprend outre les quantités appropriées de tampons et réactifs utiles pour la mise en oeuvre dudit dosage, des quantités appropriées du réactif selon la revendication 38.

**43.** Procédé de criblage de banques d'ADNc ou d'acide nucléique génomique ou de sélection de clones recombinants, caractérisé en ce que l'on révèle la présence éventuelle d'une enzyme exportée ou sécrétée à partir de clones de bactéries ou de cellules eucaryotes ayant éventuellement intégrées un plasmide, un phage, un cosmide ou un chromosome recombinant recherché comportant une séquence nucléique hybride selon l'une quelconque des revendications 1 à 16 par une réaction colorimétrique ou une sélection sur un milieu de culture approprié.

**44.** Procédé selon la revendication 43, caractérisé en ce que lorsque l'enzyme est la phosphatase alcaline, on révèle la présence de l'enzyme exportée ou sécrétée à l'aide d'un substrat approprié de ladite enzyme.

**45.** Procédé selon la revendication 43, caractérisé en ce que lorsque l'enzyme est la β-lactamase, on révèle la présence de l'enzyme exportée ou secrétée à l'aide d'un milieu contenant de l'ampicilline.

## Patentansprüche

**1.** Nukleinsäurehybridsequenz vom Typ umfassend ein Fragment, das ein in eine einen Proteinträger kodierende Sequenz insertiertes Fremdprotein kodiert, umfassend die Leadersequenz des Strukturgens eines geeigneten Proteins P1, das durch einen Mikroorganismus ausgeschieden wird oder ausscheidbar ist, ausgewählt insbesondere aus der Gruppe umfassend Bakterien und Hefen, eine Nukleinsäuresequenz, die ein NH$_2$-terminales Fragment des reifen Proteins P1 kodiert, ein Fragment, das mindestens ein funktionelles Fragment der reifen Sequenz eines geeigneten Enzyms kodiert, dadurch **gekennzeichnet** , daß eine Nukleinsäuresequenz, die ein Protein P2 kodiert, zwischen dem NH$_2$-terminalen Fragment des reifen Proteins P1 und dem Fragment, das mindestens ein funktionelles Fragment der reifen Sequenz eines geeigneten Enzyms kodiert, insertiert ist, wobei die Gesamtheit dieser Fragmente sich im Leseraster befindet und ein hybrides Protein kodiert, das gleichzeitig die Eigenschaften des Enzyms und bestimmte Eigenschaften des Proteins P2, insbesondere diejenigen der spezifischen Wechselwirkung mit einem Antikörper, einem Antigen oder einem Rezeptor besitzt.

**2.** Hybride Sequenz nach Anspruch 1, dadurch **gekennzeichnet** , daß das Enzym aus der Gruppe, umfassend insbesondere alkalische Phosphatase, saure Phosphatase, saure Glucosephosphatase, cyclische Phosphodiesterase und β-Lactamase, ausgewählt ist.

**3.** Hybride Sequenz nach Anspruch 1 oder 2, dadurch **gekennzeichnet** , daß das Fragment der Sequenz, die das Protein P2 kodiert, insbesondere aus der Gruppe umfassend Sequenzen, die Peptidhormone kodieren, Sequenzen, die ein Toxin kodieren, und Sequenzen, die ein einkettiges Fragment, das dem variablen Fragment der Immunglobuline analog ist, kodieren (rekombinante Immunglobuline), ausgewählt ist.

**4.** Hybride Sequenz nach Anspruch 3, dadurch **gekennzeichnet,** daß das Toxin vorteilhafterweise ein Neurotoxin ist.

**5.** Hybride Sequenz nach einem der Ansprüche 1 bis 4, dadurch **gekennzeichnet,** daß das Protein P1 dem Enzym identisch ist.

**6.** Hybride Sequenz nach einem der Ansprüche 1 bis 4, dadurch **gekennzeichnet,** daß das Protein P1 sich von dem Enzym unterscheidet.

**7.** Hybride Sequenz nach einem der Ansprüche 1 bis 5, dadurch **gekennzeichnet,** daß sie aus einer hybriden Sequenz besteht, umfassend aufeinanderfolgend das Leaderfragment des Strukturgens eines Enzyms, das durch einen Mikroorganismus exportiert oder ausgeschieden wird, insbesondere ausgewählt aus der Gruppe, umfassend Bakterien und Hefen, eine Nukleinsäuresequenz, die ein NH$_2$-terminales Fragment des reifen Enzyms kodiert, eine Nukleinsäuresequenz, die ein Protein P2 kodiert, anschließend ein Fragment, das mindestens ein funktionelles Fragment der reifen Sequenz des Enzyms kodiert, wobei die Gesamtheit dieser Fragmente im Leseraster vorliegt und ein Hybridprotein kodiert, das gleichzeitig die Eigenschaften des Enzyms und bestimmte Eigenschaften des Proteins, insbesondere diejenigen der spezifischen Wechselwirkung mit einem Antikörper, einem Antigen oder einem Rezeptor, besitzt.

8. Hybride Sequenz nach einem der Ansprüche 1 bis 5 oder nach Anspruch 7, dadurch **gekennzeichnet,** daß sie aufeinanderfolgend die Leadersequenz des Strukturgens der alkalischen Phosphatase, ein Fragment, das höchstens die 28 N-terminalen Aminosäuren der reifen alkalischen Phosphatase kodiert, eine DNA-Sequenz, die ein Protein P2 oder ein Fragment davon kodiert, anschließend ein Fragment, das mindestens die verbleibenden 422 C-terminalen Aminosäuren der alkalischen Phosphatase kodiert, wobei die Gesamtheit der Fragmente sich im Leseraster befindet und eine hybride DNA-Sequenz bildet, die ein Hybridprotein kodiert, das gleichzeitig die Eigenschaften der alkalischen Phosphatase, insbesondere deren enzymatische Aktivität und bestimmte Eigenschaften des Proteins P2, insbesondere die der spezifischen Wechselwirkung mit einem Antigen, einem Antikörper oder einem Rezeptor, besitzt.

9. Hybride Sequenz nach Anspruch 8, dadurch **gekennzeichnet** , daß sie aufeinanderfolgend die Nukleinsäuresequenz, die das Leaderpeptid der alkalischen Phosphatase kodiert, die Sequenz, die die 28 N-terminalen Aminosäuren der alkalischen Phosphatase kodiert, die Sequenz, die reifes Erabutoxin a (ea) kodiert, und die Sequenz, die die 422 C-terminalen Aminosäuren der alkalischen Phosphatase kodiert, umfaßt.

10. Hybride Sequenz nach Anspruch 8 oder 9, dadurch **gekennzeichnet** , daß die Sequenz, die Erabutoxin a kodiert, 192 Basenpaare und zwei zusätzliche Sau3AI-Restriktionsspaltstellen umfaßt.

11. Hybride Sequenz nach Anspruch 10, dadurch **gekennzeichnet** , daß sie die nachstehende Sequenz Nr. 1 umfaßt:

```
                                                    Arg Thr Pro
        Leaderfragment des Strukturgens von phoA-CGG ACA CCA
Glu Met Pro Val Leu Glu Asn Arg Ala Ala Gln Gly Asp Ile Thr
GAA ATG CCT GTT CTG GAA AAC CGG GCT GCT CAG GGC GAT ATT ACT
Ala Pro Gly Gly Ala Arg Arg Leu Thr Gly↓Asp Pro Arg Ile Cys
GCA CCC GGC GGT GCT CGC CGT TTA ACG GGT GAT CCC AGG ATA TGT
Phe Asn His Gln Ser Ser Gln Pro Gln Thr Thr Lys Thr Gln Ser
TTT AAC CAT CAG TCA TCG CAA CCG CAA ACC ACT AAA ACT TGT TCA
Pro Gly Glu Ser Ser Cys Tyr Asn Lys Gln Trp Ser Asp Phe Arg
CCT GGG GAG AGC TCT TGC TAT AAC AAG CAA TGG AGC GAT TTC CGT
Gly Thr Ile Ile Glu Arg Gly Cys Gly Cys Pro Thr Val Lys Pro
GGA ACT ATA ATT GAA AGG GGA TGT GGT TGC CCC ACA GTG AAG CCC
Gly Ile Lys Leu Ser Cys Cys Glu Ser Glu Val Cys Asn Asn↓Asp
GGT ATT AAA CTC AGT TGT TGC GAA TCA GAG GTC TGC AAC AAT GAT
Gln Thr
```

CAG ACT-Fragment, das mindestens die verbleibenden C-terminalen 422 Aminosäuren der alkalischen Phosphatase kodiert.

12. Hybride Sequenz nach einem der Ansprüche 1 bis 5 oder nach Anspruch 7, dadurch **gekennzeichnet,** daß sie aufeinanderfolgend die Leadersequenz des Strukturgens der alkalischen Phosphatase, ein Fragment, das die 6 N-terminalen Aminosäuren der reifen alkalischen Phosphatase kodiert, eine Nukleinsäuresequenz, die ein Protein P2 oder ein Fragment davon kodiert, anschließend ein Fragment, das die verbleibenden C-terminalen 444 Aminosäuren der alkalischen Phosphatase kodiert, umfaßt, wobei die Sequenz weiterhin insbesondere, um die Gesamtheit dieser Fragmente in das Leseraster zu bringen, stromabwärts und/oder stromaufwärts des Fragments, das das Protein P2 kodiert, ein geeignetes Nukleinsäurefragment umfaßt, wobei die hybride Sequenz im Leseraster ein hybrides Protein kodiert, das gleichzeitig die Eigenschaften des Proteins, das sich von dem Enzym unterscheidet, insbesondere die der spezifischen Wechselwirkung mit einem Antigen, einem Antikörper oder einem Rezeptor, besitzt.

13. Hybride Sequenz nach Anspruch 12, dadurch **gekennzeichnet** , daß sie aufeinanderfolgend die Leadersequenz des Strukturgens der alkalischen Phospatase, die Sequenz, die die 6 N-terminalen Aminosäuren der alkalischen Phosphatase kodiert, die Nukleinsäuresequenz, die Angiotensin 1 kodiert, ein Fragment umfassend die Sequenz AGG G, die erlaubt, das Gen der alkalischen Phosphatase in das Leseraster zu bringen, anschließend ein Fragment, das mindestens die 444 verbleibenden C-terminalen Aminosäuren der alkalischen Phosphatase kodiert, umfaßt.

**14.** Hybride Sequenz nach Anspruch 13, dadurch **gekennzeichnet** , daß sie die nachstehende Sequenz Nr. 2 umfaßt:

```
-21
GTG AAA CAA AGC ACT ATT GCA CTG GCA CTC TTA CCG TTA CTG
Met lys gln ser thr ile ala leu ala leu leu pro leu leu

                                    -1  +1                  +6
TTT ACC CCT GTG ACA AAA GCC CGG ACA CCA GAA ATG ccc GAT
phe thr pro val thr lys ala arg thr pro glu met pro asp

                                                +7
CGT GTC TAT ATT CAC CCG TTT CAC CTT Agg gTT CTG GAA AAC
arg val tyr ile his pro phe his leu arg val leu glu asn

CGG GCT GCT CAG GGC GAT ATT ACT GCA CCC GGC GGT GCT CGC
arg ala ala gln gly asp ile thr ala pro gly gly ala arg

CGT TTA ACG ...
arg leu thr ...
        .
        .
        .       +450
... CTG AAA TAA
... leu lys stop
```

**15.** Hybride Sequenz nach Anspruch 12, dadurch **gekennzeichnet** , daß sie aufeinanderfolgend die Leadersequenz des Strukturgens der alkalischen Phosphatase, die Sequenz, die die 6 N-terminalen Aminosäuren der alkalischen Phosphatase kodiert, ein Fragment, umfassend die Sequenz GAT CCC, die Nukleinsäuresequenz, die Erabutoxin a kodiert, ein Fragment, umfassend die Sequenz GAT C, wobei das Fragment erlaubt, das Gen der alkalischen Phosphatase in das Leseraster zu bringen, anschließend ein Fragment, das mindestens die 444 verbleibenden C-terminalen Aminosäuren der alkalischen Phosphatase kodiert, umfaßt.

**16.** Sequenz nach Anspruch 15, dadurch **gekennzeichnet** , daß sie die nachstehende Sequenz Nr. 3 umfaßt:

```
-21
GTG AAA CAA AGC ACT ATT GCA CTG GCA CTC TTA CCG TTA CTG
Met lys gln ser thr ile ala leu ala leu leu pro leu leu

                            -1 +1                  +6
TTT ACC CCT GTG ACA AAA GCC CGG ACA CCA GAA ATG CCC GAT
phe thr pro val thr lys ala arg thr pro glu met pro asp

CCC AGG ATA TGT TTT AAC CAT CAG TCA TCG CAA CCG CAA ACC
pro arg ile cys phe asn his gln ser ser gln pro gln thr

ACT AAA ACT TGT TCA CCT GGG GAG AGC TCT TGC TAT AAC AAG
thr lys thr cys ser pro gly glu ser ser cys tyr asn lys

CAA TGG AGC GAT TTC CGT GGA ACT ATA ATT GAA AGG GGA TGT
gln trp ser asp phe arg gly thr ile ile glu arg gly cys

GGT TGC CCC ACA GTG AAG CCC GGT ATT AAA CTC AGT TGT TGC
gly cys pro thr val lys pro gly ile lys leu ser cys cys

                                        +7
GAA TCA GAG GTC TGC AAC AAT GAT Cgg gTT CTG GAA AAC CGG
glu ser glu val cys asn asn asp arg val leu glu asn arg

GCT GCT CAG GGC GAT ATT ACT GCA CCC GGC GGT GCT CGC CGT
ala ala gln gly asp ile thr ala pro gly gly ala arg arg

TTA ACG GGT GAT CAG ACT GCC ...
leu thr gly asp gln thr ala ...

  .

  .
  .      +450
... CTG AAA TAA
... leu lys stop
```

**17.** Protein, bestehend aus einer hybriden Sequenz, umfassend ein Fragment eines Fremdproteins, das in einen Proteinträger insertiert ist, umfassend ein Fragment eines Proteins P1, das durch einen Mikroorganismus exportiert oder sezerniert wird, insbesondere ausgewählt aus der Gruppe umfassend Bakterien und Hefen, und ein funktionelles Fragment eines geeigneten Enzyms, wobei die Sequenz dadurch gekennzeichnet ist, daß ein geeignetes Protein P2 zwischen das Fragment des Proteins P1, das durch einen Mikroorganismus exportiert oder sezerniert wird, und das funktionelle Fragment eines geeigneten Enzyms insertiert ist, wobei das hybride Protein gleichzeitig die Eigenschaften des Enzyms und bestimmte Eigenschaften des Proteins P2, insbesondere die der spezifischen Wechselwirkung mit einem Antikörper, einem Antigen oder einem Rezeptor, besitzt.

**18.** Hybrides Protein nach Anspruch 17, dadurch **gekennzeichnet** , daß das Protein P1 sich von dem Enzym unterscheidet.

**19.** Hybrides Protein nach Anspruch 17, dadurch **gekennzeichnet** , daß das Protein P1 dem Enzym identisch ist.

**20.** Hybrides Protein nach Anspruch 17 oder 19, dadurch **gekennzeichnet,** daß es ein Enzym umfaßt, das durch einen Mikroorganismus exportiert oder sezerniert wird, ausgewählt insbesondere aus der Gruppe, umfassend Bakterien und Hefen, in das ein geeignetes Protein P2 insertiert ist.

**21.** Hybrides Protein nach Anspruch 17 oder 19, dadurch **gekennzeichnet,** daß es aufeinanderfolgend ein Fragment eines Enzyms, das durch einen Mikroorganismus exportiert oder sezerniert wird, insbesondere ausgewählt aus der Gruppe umfassend Bakterien und Hefen, ein geeignetes Protein P2, anschließend die vollständige reife Sequenz des Enzyms umfaßt.

**22.** Hybrides Protein nach einem der Ansprüche 17 bis 21, dadurch **gekennzeichnet,** daß es ein Protein P2 enthält, ausgewählt insbesondere aus der Gruppe, umfassend Peptidhormone, einkettige Fragmente, die den variablen Domänen der Immunglobuline analog sind, und Toxine, insertiert in ein Enzym, ausgewählt aus der Gruppe, umfassend insbesondere alkalische Phosphatase, saure Phosphatase, saure Glucosephosphatase, cyclische Phosphodiesterase und ß-Lactamase.

**23.** Hybrides Protein nach Anspruch 22, dadurch **gekennzeichnet** , daß es vorteilhafterweise ein Neurotoxin, insertiert in die alkalische Phosphatase, umfaßt.

**24.** Hybrides Protein nach Anspruch 22 oder 23, dadurch **gekennzeichnet,** daß das Neurotoxin Erabutoxin a (ea) ist, wobei das hybride Protein die von den Formeln I oder III, gemäß Anspruch 12 oder 16, abgeleitete Sequenz besitzt.

**25.** Hybrides Protein nach Anspruch 22, dadurch **gekennzeichnet** , daß es vorteilhafterweise Angiotensin 1, insertiert in die alkalische Phosphatase und mit der von Formel II nach Anspruch 14 abgeleiteten Sequenz, besitzt.

**26.** Familie von Expressionsvektoren und/oder Klonierungsvektoren eines hybriden Proteins nach einem der Ansprüche 17 bis 25, dadurch **gekennzeichnet,** daß jeder Vektor ein Expressionssystem einschließt, umfassend

- einen geeigneten Promotor
- eine Ribosomenbindungsstelle
- eine Nukleinsäuresequenz, umfassend die Leadersequenz des Strukturgens eines Proteins P1, eine Sequenz, die ein $NH_2$-terminales Fragment des reifen Proteins P1 kodiert, eine Sequenz, die ein Protein P2, das in die natürliche Restriktionsspaltstelle insertiert ist, kodiert, und die Sequenz, die ein reifes extracytoplasmatisches Enzym oder ein Fragment davon kodiert, wobei die Nukleinsäuresequenz einer erfindungsgemäßen hybriden Sequenz entspricht, und
- einen Transkriptionsterminator,
wobei das Expressionssystem in eine geeignete genetische Struktur, insbesondere ausgewählt aus der Gruppe, umfassend Plasmide, Phagen, Cosmide oder geeignete Chromosomen, insertiert ist.

**27.** Vektor nach Anspruch 26, dadurch **gekennzeichnet,** daß die genetische Struktur ein Plasmid ist und das Protein P1 dem Enzym identisch ist.

**28.** Vektor nach Anspruch 26 oder 27, dadurch **gekennzeichnet,** daß, wenn P2 vorteilhafterweise Erabutoxin a ist, man ein Plasmid mit der Bezeichnung pEP1726 erhält, das die folgenden Eigenschaften besitzt:

- es umfaßt 6,1 kb
- es wird durch Ligierung des Plasmids pJC2431, enthaltend das Strukturgen der alkalischen Phosphatase und das Ampicillinresistenzgen ($Ap^R$) trägt, wobei das Plasmid an der BclI-Spaltstelle bei Kodon 28 des Strukturgens der alkalischen Phosphatase mit dem Sau3AI-Sau3AI-Fragment von 192 Basenpaaren, die Erabutoxin a kodieren, linearisiert wurde

**29.** Familie von Expressionsvektoren und/oder Klonierungsvektoren eines hybriden Proteins nach einem der Ansprüche 17 bis 25, dadurch **gekennzeichnet,** daß jeder Vektor ein Expressionssystem einschließt, umfassend:

- einen geeigneten Promotor
- eine Ribosomenbindungsstelle
- eine Nukleinsäuresequenz, umfassend die Leadersequenz des Strukturgens eines Proteins P1, eine Sequenz, die ein $NH_2$-terminales Fragment des reifen Proteins P1 kodiert, die Sequenz, die ein reifes extracytoplasmatisches Enzym oder ein Fragment davon kodiert, und eine oder mehrere, einzigartige Restriktionsspaltstellen, die geeignet sind, eine Sequenz aufzunehmen, die ein Protein P2 kodiert, die an der Verbindung zwischen dem Fragment der Sequenz, die das Protein P1 kodiert, und der Sequenz, die das Enzym kodiert, angebracht sind, und
- einen Transkriptionsterminator, wobei das Expressionssystem in eine geeignete, genetische Struktur, ausgewählt insbesondere aus der Gruppe, umfassend Plasmide, Phagen, Cosmide oder geeignete Chromosomen, insertiert ist.

**30.** Vektor nach Anspruch 29, dadurch **gekennzeichnet,** daß mindestens eine der eingebrachten, einzigartigen Restriktionsspaltstellen eine Verschiebung des Gens des Enzyms vom Leseraster herbeiführt, wobei die Einbringung des Gens in das Leseraster durch Einbringen der Sequenz des Proteins P2 gewährleistet wird.

31. Vektor nach Anspruch 29 oder 30, dadurch **gekennzeichnet,** daß man ein Plasmid mit der Bezeichnung pLIP1 erhält, das die folgenden Eigenschaften besitzt:

   - es umfaßt etwa 5,9 kb und
   - es wird durch gerichtete Mutagenese des phoA-Gens, das in dem Plasmid pJC2431 vorhanden ist, derart, daß eine einzigartige Restriktionsspaltstelle SmaI, entsprechend der Position +6 des reifen Proteins, eingebracht wird, erhalten.

32. Mikroorganismus, erhalten durch genetische Transformation, dadurch **gekennzeichnet,** daß er durch geeignete Modifikation eines geeigneten Stammes von E. coli durch einen Vektor nach einem der Ansprüche 26 bis 31 erhalten wird.

33. Mikroorganismus nach Anspruch 32, dadurch **gekennzeichnet,** daß er vorteilhafterweise ein durch den Vektor pEP1726 transformierter Stamm CC118 von E. coli ist.

34. Mikroorganismus nach Anspruch 33 mit der Bezeichnung SEP 1726, dadurch **gekennzeichnet,** daß er am 2. Juni 1989 unter der Hinterlegungsnummer I-862 bei der Collection Nationale des Cultures de Microorganismes des Institut Pasteur hinterlegt wurde.

35. Mikroorganismus nach Anspruch 32, dadurch **gekennzeichnet,** daß er vorteilhafterweise ein durch das Plasmid pLIP1 transformierter Stamm CC118 von E. coli ist.

36. Mikroorganismus nach Anspruch 35, dadurch **gekennzeichnet,** daß er am 7. Juni 1990 unter der Hinterlegungsnummer I-954 bei der Collection Nationale des Cultures de Microorganismes im Institut Pasteur hinterlegt wurde.

37. Verfahren zur Expression eines hybriden Proteins nach einem der Ansprüche 17 bis 25, dadurch **gekennzeichnet,** daß man einen Expressionsvektor nach einem der Ansprüche 26 bis 31 in einen Mikroorganismus nach einem der Ansprüche 32 bis 36 einsetzt.

38. Diagnostisches Reagens, dadurch **gekennzeichnet,** daß es aus einem hybriden Protein nach einem der Ansprüche 17 bis 25 besteht.

39. Verfahren zur immunenzymologischen Bestimmung von Proteinen, dadurch **gekennzeichnet,** daß man in einer biologischen Flüssigkeit vorhandene Proteine dadurch nachweist, daß man die biologische Flüssigkeit mit dem Diagnosereagens nach Anspruch 38 in Kontakt bringt, und daß die Anwesenheit des Reagenses in Form eines Komplexes oder in freier Form durch eine geeignete colorimetrische Reaktion nachgewiesen wird.

40. Bestimmungsverfahren nach Anspruch 39, dadurch **gekennzeichnet,** daß die Proteine Antigene sind.

41. Bestimmungsverfahren nach Anspruch 39, dadurch **gekennzeichnet,** daß die Proteine Antikörper sind.

42. Gebrauchsfertiges Kit zur Durchführung einer enzym-immunologischen Bestimmung nach einem der Ansprüche 39 bis 41, dadurch **gekennzeichnet,** daß es weiterhin geeignete Mengen an Puffern und Reagentien, die zur Durchführung der Bestimmung nützlich sind, geeignete Mengen des Reagenses nach Anspruch 38 umfaßt.

43. Verfahren zur Absuchung von Banken von cDNA oder genomischer Nukleinsäure oder zur Selektion von rekombinanten Clonen, dadurch **gekennzeichnet,** daß man die eventuelle Anwesenheit eines Enzyms, das aus Clonen von Bakterien oder eucaryotischen Zellen exportiert oder sezerniert wird, in die gegebenenfalls ein gewünschtes Plasmid, ein gewünschter Phage, ein gewünschtes Cosmid oder ein gewünschtes rekombinantes Chromosom integriert ist, umfassend eine hybride Nukleinsäuresequenz nach einem der Ansprüche 1 bis 16, durch eine colorimetrische Reaktion oder eine Selektion auf einem geeigneten Kulturmedium nachweist.

44. Verfahren nach Anspruch 43, dadurch **gekennzeichnet,** daß, wenn das Enzym die alkalische Phosphatase ist, man die Anwesenheit des exportierten oder sezernierten Enzyms mit Hilfe eines geeigneten Substrats des Enzyms nachweist.

**45.** Verfahren nach Anspruch 43, dadurch **gekennzeichnet,** daß, wenn das Enzym die β-Lactamase ist, man die Anwesenheit des exportierten oder sezernierten Enzyms mit Hilfe eines ampicillinhaltigen Mediums nachweist.

**Claims**

**1.** hybrid nucleic acid sequence, of the type comprising a fragment coding for a foreign protein inserted into a sequence coding for a carrier protein comprising the leader sequence of the structural gene for a suitable protein P1 exported or secretable by a microorganism, in particular chosen from the group which comprises bacteria and yeasts, a nucleic acid sequence coding for an NH$_2$-terminal fragment of the said mature protein P1, and a fragment coding for at least one functional fragment of the mature sequence of a suitable enzyme, which sequence is characterized in that a nucleic acid sequence coding for a protein P2 is inserted between the said NH$_2$-terminal fragment of the said mature protein P1 and the said fragment coding for at least one functional fragment of the mature sequence of a suitable enzyme, this set of fragments being in a single reading frame and coding for a hybrid protein possessing simultaneously the properties of the enzyme and some properties of the said protein P2, in particular that of interacting specifically with an antibody, an antigen or a receptor.

**2.** Hybrid sequence according to Claim 1, characterized in that the enzyme is chosen from the group which comprises, in particular, alkaline phosphatase, acid phosphatase, acid glucose phosphatase, cyclic phosphodiesterase and β-lactamase.

**3.** Hybrid sequence according to Claim 1 or Claim 2, characterized in that the fragment of sequence coding for the protein P2 is, in particular, chosen from the group which comprises the sequences coding for peptide hormones, the sequences coding for a toxin and the sequences coding for a single-chain fragment analogous to the variable fragment of immunoglobulins (recombinant immunoglobulins).

**4.** Hybrid sequence according to Claim 3, characterized in that the toxin is advantageously a neurotoxin.

**5.** Hybrid sequence according to any one of Claims 1 to 4, characterized in that the protein P1 is identical to the enzyme.

**6.** Hybrid sequence according to any one of Claims 1 to 4, characterized in that the protein P1 is different from the enzyme.

**7.** Hybrid sequence according to any one of Claims 1 to 5, characterized in that it consists of a hybrid sequence comprising successively the leader fragment of the structural gene of an enzyme exported or secreted by a microorganism, in particular chosen from the group which comprises bacteria and yeasts, a nucleic acid sequence coding for an NH$_2$-terminal fragment of the said mature enzyme, a nucleic acid sequence coding for a protein P2 and then a fragment coding for at least one functional fragment of the mature sequence of the said enzyme, this set of fragments being in a single reading frame and coding for a hybrid protein possessing simultaneously the properties of the enzyme and some properties of the said protein, in particular that of interacting specifically with an antibody, an antigen or a receptor.

**8.** Hybrid sequence according to any one of Claims 1 to 5 or according to Claim 7, characterized in that it comprises successively the leader sequence of the structural gene for alkaline phosphatase, a fragment coding for, at most, the 28 N-terminal amino acids of mature alkaline phosphatase, a DNA sequence coding for a protein P2 or a fragment of the latter, and then a fragment coding for, at least, the remaining 422 C-terminal amino acids of alkaline phosphatase, this set of fragments being in a single reading frame and forming a hybrid DNA sequence coding for a hybrid protein possessing simultaneously the properties of alkaline phosphatase, especially its enzymatic activity, and some of the properties of the protein P2, in particular that of interacting specifically with an antigen, an antibody or a receptor.

**9.** Hybrid sequence according to Claim 8, characterized in that it comprises successively the nucleic acid sequence coding for the leader peptide of alkaline phosphatase, the sequence coding for the 28 N-terminal amino acids of alkaline phosphatase, the sequence coding for mature erabutoxin a (ea) and the sequence coding for the 422 C-terminal amino acids of alkaline phosphatase.

**10.** Hybrid sequence according to Claim 8 or Claim 9, characterized in that the sequence coding for erabutoxin a comprises 192 base pairs and two additional Sau3AI restriction sites.

**11.** Hybrid sequence according to Claim 10, characterized in that it comprises the sequence No. 1 below:

```
                                               Arg Thr Pro
Leader fragment of the structural gene for phoA-CGG ACA CCA
Glu Met  Pro Val Leu Glu Asn Arg Ala Ala Gln Gly Asp Ile Thr
GAA ATG  CCT GTT CTG GAA AAC CGG GCT GCT CAG GGC GAT ATT ACT
Ala Pro  Gly Gly Ala Arg Arg Leu Thr Gly↓Asp Pro Arg Ile Cys
GCA CCC  GGC GGT GCT CGC CGT TTA ACG GGT GAT CCC AGG ATA TGT
Phe Asn  His Gln Ser Ser Gln Pro Gln Thr Thr Lys Thr Gln Ser
TTT AAC  CAT CAG TCA TCG CAA CCG CAA ACC ACT AAA ACT TGT TCA
Pro Gly  Glu Ser Ser Cys Tyr Asn Lys Gln Trp Ser Asp Phe Arg
CCT GGG  GAG AGC TCT TGC TAT AAC AAG CAA TGG AGC GAT TTC CGT
Gly Thr  Ile Ile Glu Arg Gly Cys Gly Cys Pro Thr Val Lys Pro
GGA ACT  ATA ATT GAA AGG GGA TGT GGT TGC CCC ACA GTG AAG CCC
Gly Ile  Lys Leu Ser Cys Cys Glu Ser Glu Val Cys Asn Asn↓Asp
GGT ATT  AAA CTC AGT TGT TGC GAA TCA GAG GTC TGC AAC AAT GAT
Gln Thr
```

CAG ACT-fragment coding for at least the remaining 422 C-terminal amino acids of alkaline phosphatase.

**12.** Hybrid sequence according to any one of Claims 1 to 5 or according to Claim 7, characterized in that it comprises successively the leader sequence of the structural gene for alkaline phosphatase, a fragment coding for the 6 N-terminal amino acids of mature alkaline phosphatase, a nucleic acid sequence coding for a protein P2 or a fragment of the latter, and then a fragment coding for the remaining 444 C-terminal amino acids of alkaline phosphatase, which sequence comprises, in addition, in particular in order to place this set of fragments in a single reading frame, downstream and/or upstream from the fragment coding for the protein P2, a suitable nucleic acid fragment, which hybrid sequence, in a single reading frame, codes for a hybrid protein possessing simultaneously the properties of alkaline phosphatase and some of the properties of the protein different from the enzyme, in particular that of interacting specifically with an antigen, an antibody or a receptor.

**13.** Hybrid sequence according to Claim 12, characterized in that it comprises successively the leader sequence of the structural gene for alkaline phosphatase, the sequence coding for the 6 N-terminal amino acids of alkaline phosphatase, the nucleic acid sequence coding for angiotensin I, a fragment comprising the sequence AGG G and which enables the alkaline phosphatase gene to be shifted back into frame, and then a fragment coding for, at least, the remaining 444 C-terminal amino acids of alkaline phosphatase.

**14.** Hybrid sequence according to Claim 13, characterized in that it comprises the sequence No. 2 below:

```
-21
GTG AAA CAA AGC ACT ATT GCA CTG GCA CTC TTA CCG TTA CTG
Met lys gln ser thr ile ala leu ala leu leu pro leu leu

                              -1 +1                  +6
TTT ACC CCT GTG ACA AAA GCC CGG ACA CCA GAA ATG ccc GAT
phe thr pro val thr lys ala arg thr pro glu met pro asp

                                        +7
CGT GTC TAT ATT CAC CCG TTT CAC CTT Agg gTT CTG GAA AAC
arg val tyr ile his pro phe his leu arg val leu glu asn

CGG GCT GCT CAG GGC GAT ATT ACT GCA CCC GGC GGT GCT CGC
arg ala ala gln gly asp ile thr ala pro gly gly ala arg

CGT TTA ACG ...
arg leu thr ...

.
.
.            +450
... CTG AAA TAA
... leu lys stop
```

**15.** Hybrid sequence according to Claim 12, characterized in that it comprises successively the leader sequence of the structural gene for alkaline phosphatase, the sequence coding for the 6 N-terminal amino acids of alkaline phosphatase, a fragment comprising the sequence GAT CCC, the nucleic acid sequence coding for erabutoxin a, a fragment comprising the sequence GAT C, which fragment enables the alkaline phosphatase gene to be shifted back into frame, and then a fragment coding for, at least, the remaining 444 C-terminal amino acids of alkaline phosphatase.

**16.** Sequence according to Claim 15, characterized in that it comprises the sequence No. 3 below:

```
-21
GTG AAA CAA AGC ACT ATT GCA CTG GCA CTC TTA CCG TTA CTG
Met lys gln ser thr ile ala leu ala leu leu pro leu leu

                        -1 +1                       +6
TTT ACC CCT GTG ACA AAA GCC CGG ACA CCA GAA ATG ccc GAT
phe thr pro val thr lys ala arg thr pro glu met pro asp

CCC AGG ATA TGT TTT AAC CAT CAG TCA TCG CAA CCG CAA ACC
pro arg ile cys phe asn his gln ser ser gln pro gln thr

ACT AAA ACT TGT TCA CCT GGG GAG AGC TCT TGC TAT AAC AAG
thr lys thr cys ser pro gly glu ser ser cys tyr asn lys

CAA TGG AGC GAT TTC CGT GGA ACT ATA ATT GAA AGG GGA TGT
gln trp ser asp phe arg gly thr ile ile glu arg gly cys

GGT TGC CCC ACA GTG AAG CCC GGT ATT AAA CTC AGT TGT TGC
gly cys pro thr val lys pro gly ile lys leu ser cys cys

                                    +7
GAA TCA GAG GTC TGC AAC AAT GAT Cgg gTT CTG GAA AAC CGG
glu ser glu val cys asn asn asp arg val leu glu asn arg

GCT GCT CAG GGC GAT ATT ACT GCA CCC GGC GGT GCT CGC CGT
ala ala gln gly asp ile thr ala pro gly gly ala arg arg

TTA ACG GGT GAT CAG ACT GCC ...
leu thr gly asp gln thr ala ...
.
.
.        +450
... CTG AAA TAA
... leu lys stop
```

**17.** Protein, consisting of a hybrid sequence comprising a fragment of foreign protein inserted into a carrier protein comprising a fragment of a protein P1 exported or secreted by a microorganism, in particular chosen from the group which comprises bacteria and yeasts, and a functional fragment of a suitable enzyme, which sequence is characterized in that a suitable protein P2 is inserted between the said fragment of protein P1 exported or secreted by a microorganism and the said functional fragment of a suitable enzyme, the said hybrid protein possessing simultaneously the properties of the enzyme and some properties of the protein P2, in particular that of interacting specifically with an antibody, an antigen or a receptor.

**18.** Hybrid protein according to Claim 17, characterized in that the protein P1 is different from the enzyme.

**19.** Hybrid protein according to Claim 17, characterized in that the protein P1 is identical to the enzyme.

**20.** Hybrid protein according to Claim 17 or Claim 19, characterized in that it comprises an enzyme exported or secreted by a microorganism, in particular chosen from the group which comprises bacteria and yeasts, into which a suitable protein P2 is inserted.

21. Hybrid protein according to Claim 17 or Claim 19, characterized in that it comprises successively a fragment of an enzyme exported or secreted by a microorganism, in particular chosen from the group which comprises bacteria and yeasts, a suitable protein P2 and then the complete mature sequence of the said enzyme.

22. Hybrid protein according to any one of Claims 17 to 21, characterized in that it contains a protein P2, in particular chosen from the group which comprises peptide hormones, single-chain fragments analogous to the variable domains of immunoglobulins and toxins, inserted into an enzyme chosen from the group which comprises, in particular, alkaline phosphatase, acid phosphatase, acid glucose phosphatase, cyclic phosphodiesterase and β-lactamase.

23. Hybrid protein according to Claim 22, characterized in that it advantageously comprises a neurotoxin inserted into alkaline phosphatase.

24. Hybrid protein according to Claim 22 or Claim 23, characterized in that the neurotoxin is erabutoxin a (ea), which hybrid protein possesses the sequence deduced according to the sequence No. 1 or No. 3 according to Claim 12 or Claim 16.

25. Hybrid protein according to Claim 22, characterized in that it advantageously comprises angiotensin I inserted into alkaline phosphatase, and possesses the sequence deduced according to the sequence No. 2 according to Claim 14.

26. Family of vectors for the expression and/or cloning of a hybrid protein according to any one of Claims 17 to 25, characterized in that each vector includes an expression system comprising:

- a suitable promoter,
- a ribosome-binding site,
- a nucleic acid sequence containing the leader sequence of the structural gene for a protein P1, a sequence coding for an $NH_2$-terminal fragment of the said mature protein P1, a sequence coding for a protein P2 inserted at a natural restriction site and the sequence coding for a mature extracytoplasmic enzyme or a fragment of the latter, which nucleic acid sequence corresponds to a hybrid sequence according to the invention, and
- a transcription terminator,

    which expression system is inserted into a suitable genetic structure, in particular chosen from the group which comprises plasmids, phages, cosmids or suitable chromosomes.

27. Vector according to Claim 26, characterized in that the genetic structure is a plasmid and the protein P1 is identical to the enzyme.

28. Vector according to Claim 26 or Claim 27, characterized in that, when P2 is advantageously erabutoxin a, a plasmid designated pEP1726 is obtained, possessing the following properties:

- it comprises 6.1 kb;
- it is obtained by ligation of plasmid pJC2431 carrying the structural gene for alkaline phosphatase and the gene for resistance to ampicillin ($Ap^R$) the said plasmid being linearized at the Bc1I site, at codon 28 of the said structural gene for alkaline phosphatase - with the 192-base pair Sau3AI-Sau3AI fragment coding for erabutoxin a.

29. Family of vectors for the expression and/or cloning of a hybrid protein according to any one of Claims 17 to 25, characterized in that each vector includes an expression system comprising:

- a suitable promoter,
- a ribosome-binding site,
- a nucleic acid sequence containing the leader sequence of the structural gene for a protein P1, a sequence coding for an $NH_2$-terminal fragment of the said mature protein P1, the sequence coding for a mature extracytoplasmic enzyme or a fragment of the latter and one or more unique restriction sites, capable of receiving a sequence coding for a protein P2, situated at the junction between the fragment of sequence coding for the protein P1 and the sequence coding for the enzyme, and
- a transcription terminator,
  which expression system is inserted into a suitable genetic structure, in particular chosen from the group which comprises plasmids, phages, cosmids or suitable chromosomes.

30. Vector according to Claim 29, characterized in that at least one of the unique restriction sites introduced causes a shifting of the gene for the enzyme out of phase, the shifting of the said gene back into phase being effected by the introduction of the sequence for the protein P2.

31. Vector according to Claim 29 or Claim 30, characterized in that a plasmid designated pLIP1 is obtained, possessing the following properties:

- it comprises approximately 5.9 kb, and
- it is obtained by directed mutagenesis of the phoA gene carried by plasmid pJC2431, so as to introduce a unique SmaI restriction site, corresponding to position +6 of the mature protein.

32. Microorganism obtained by genetic transformation, characterized in that it is obtained by suitable modification of a suitable strain of E. coli with a vector according to any one of Claims 26 to 31.

33. Microorganism according to Claim 32, characterized in that it is advantageously an E. coli strain CC118 transformed by the vector pEP1726.

34. Microorganism according to Claim 33, designated SEP 1726, characterized in that it was deposited on the date 2 June 1989 under number I-862 with the Collection Nationale des Cultures de Microorganismes [National Collection of Microorganism Cultures] held by the Pasteur Institute.

35. Microorganism according to Claim 32, characterized in that it is advantageously an E. coli strain CC118 transformed by plasmid pLIP1.

36. Microorganism according to Claim 35, characterized in that it was deposited on the date 7 June 1990 under number I-954 with the Collection Nationale des Cultures de Microorganismes [National Collection of Microorganism Cultures] held by the Pasteur Institute.

37. Process for the expression of a hybrid protein according to any one of Claims 17 to 25, characterized in that it employs an expression vector according to any one of Claims 26 to 31, in a microorganism according to any one of Claims 32 to 36.

38. Diagnostic reagent, characterized in that it consists of a hybrid protein according to any one of Claims 17 to 25.

39. Method for the immunoenzymological assay of proteins, characterized in that it consists in detecting proteins present in a biological fluid by bringing the said biological fluid into contact with the diagnostic reagent according to Claim 38 and in that the presence of the said reagent in the form of a complex or in free form is visualized by a suitable colorimetric reaction.

40. Assay method according to Claim 39, characterized in that the said proteins are antigens.

41. Assay method according to Claim 39, characterized in that the said proteins are antibodies.

42. Ready-to-use kit for carrying out an immunoenzymological assay according to any one of Claims 39 to 41, characterized in that it comprises, apart from the appropriate quantities of buffers and reagents useful for carrying out the said assay, suitable quantities of the reagent according to Claim 38.

43. Method for screening libraries of cDNA or of genomic nucleic acid, or for the selection of recombinant clones, characterized in that the possible presence of an enzyme exported or secreted from clones of bacteria or of eukaryotic cells which have, where appropriate, integrated a plasmid, a phage, a cosmid or a recombinant chromosome under investigation containing a hybrid nucleic acid sequence according to any one of Claims 1 to 16 is visualized by a colorimetric reaction or a selection on a suitable culture medium.

44. Method according to Claim 43, characterized in that, when the enzyme is alkaline phosphatase, the presence of the exported or secreted enzyme is visualized using a suitable substrate for the said enzyme.

45. Method according to Claim 43, characterized in that, when the enzyme is β-lactamase, the presence of the exported or secreted enzyme is visualized using a medium containing ampicillin.

FIG. 1

FIG.2

FIG.3